# EUROPÄISCHE PATENTSCHRIFT

(11) **EP 1 551 873 B1**
(45) Veröffentlichungstag und Bekanntmachung des Hinweises auf die Patenterteilung: **15.05.2013**
(21) Anmeldenummer: 03807832.5
(22) Anmeldetag: 02.10.2003
(51) Int. Cl.: C07K 14/705, A61K 31/11

(54) **AGONISTEN UND ANTAGONISTEN DES HUMANEN DUFTSTOFFREZEPTORS OR17-4 SOWIE VERWENDUNGEN DAVON**
AGONISTS AND ANTAGONISTS OF THE HUMAN ODORANT RECEPTOR OR17-4 AND USES THEREOF
AGONISTES ET ANTAGONISTES DE RECEPTEUR D'ODEURS HUMAIN OR17-4 ET LEUR UTILISATION

(30) Priorität: 04.10.2002 DE 10246329
(43) Veröffentlichungstag der Anmeldung: 13.07.2005
(73) Patentinhaber: Symrise AG, 37603 Holzminden (DE)
(72) Erfinder: HATT, Hanns, 44789 Bochum (DE)
(74) Vertreter: Eisenführ, Speiser & Partner
(86) Internationale Anmeldenummer: PCT/EP2003/010915
(87) Internationale Veröffentlichungsnummer: WO 2004/033496

(56) Entgegenhaltungen:
- NEKRASOVA ELINA ET AL: "Overexpression, solubilization and purification of rat and human olfactory receptors" EUROPEAN JOURNAL OF BIOCHEMISTRY, Bd. 238, Nr. 1, 1996, Seiten 28-37, XP001157260 ISSN: 0014-2956 & DATABASE EMBL [Online] EBI; 18. November 1993 (1993-11-18) PARMENTIER M.: "H.sapiens HGMP07E gene for olfactory receptor." Database accession no. X65857
- KIEFER HANS ET AL: "Expression of an olfactory receptor in Escherichia coli: Purification, reconstitution, and ligand binding" BIOCHEMISTRY, Bd. 35, Nr. 50, 1996, Seiten 16077-16084, XP002130409 ISSN: 0006-2960 & DATABASE EMBL [Online] EBI; 22. August 1996 (1996-08-22) RAMING K. ET AL.: "R. norvegicus mRNA for olfactory receptor." Database accession no. Y07557
- WETZEL CHRISTIAN H ET AL: "Specificity and sensitivity of a human olfactory receptor functionally expressed in human embryonic kidney 293 cells and Xenopus laevis oocytes" JOURNAL OF NEUROSCIENCE, Bd. 19, Nr. 17, 1. September 1999 (1999-09-01), Seiten 7426-7433, XP002178954 ISSN: 0270-6474 in der Anmeldung erwähnt
- KRAUTWURST DIETMAR ET AL: "Identification of ligands for olfactory receptors by functional expression of a receptor library" CELL, Bd. 95, Nr. 7, 23. Dezember 1998 (1998-12-23), Seiten 917-926, XP002268732 ISSN: 0092-8674 in der Anmeldung erwähnt
- GLUSMAN GUSTAVO ET AL: "Sequence, structure, and evolution of a complete human olfactory receptor gene cluster" GENOMICS, Bd. 63, Nr. 2, 15. Januar 2000 (2000-01-15), Seiten 227-245, XP002178957 ISSN: 0888-7543
- ZHAO H ET AL: "Vertebrate odorant receptors" CMLS CELLULAR AND MOLECULAR LIFE SCIENCES, Bd. 56, Nr. 7-8, 15. November 1999 (1999-11-15), Seiten 647-659, XP002268734 ISSN: 1420-682X
- SPEHR MARC ET AL: "Identification of a testicular odorant receptor mediating human sperm chemotaxis." SCIENCE (WASHINGTON D C), Bd. 299, Nr. 5615, 28. März 2003 (2003-03-28), Seiten 2054-2058, XP002268735 ISSN: 0036-8075 (ISSN print)

### SEQUENCE LISTING

<110> Symrise GmbH & Co. KG
<120> Duftstoffrezeptoren
<130> 610107PC
<160> 12
<170> PatentIn version 3.1
<210> 1.
   <211> 32
   <212> DNA
   <213> Artificial Sequence
<220>
   <223> Primer pOR17-4 upstream-1
<400> 1
   tccaccatgg atggaggcaa ccagagtgaa gg 32
<210> 2
   <211> 28
   <212> DNA
   <213> Artificial Sequence
<220>
   <223> primer pOR17-4 downstream-1
<400> 2
   atagtctcca ctttaatgct gtctttcc 28
<210> 3
   <211> 982
   <212> DNA
   <213> Artificial Sequence
<220>
   <223> hOR17-4 PCR
<400> 3
   tccaccatgg atggaggcaa ccagagtgaa ggttcagagt tccttctcct ggggatgtca    60
   gagagtcctg agcagcagca gatcctgttt tggatgttcc tgtccatgta cctggtcacg   120
   gtggtgggaa atgtgctcat catcctggcc atcagctctg attcccgcct gcacaccccc   180
   gtgtacttct tcctggccaa cctctccttc actgacctct tctttgtcac caacacaatc   240
   cccaagatgc tggtgaacct ccagtcccat aacaaagcca tctcctatgc agggtgtctg   300
   acgcagctct acttcctggt ctccttggtg gccctggaca acctcatcct ggctgtgatg   360
   gcatatgacc gctatgtggc catctgctgc cccctccact acaccacagc catgagccct   420
   aagctctgta tcttactcct ttccttgtgt tgggtcctat ccgtcctcta tggcctcata   480
   cacaccctcc tcatgaccag agtgaccttc tgtgggtcac gaaaaatcca ctacatcttc   540
   tgtgagatgt atgtattgct gaggatggca tgttccaaca ttcagattaa tcacacagtg   600
   ctgattgcca caggctgctt catcttcctc attccctttg gattcgtgat catttcctat   660
   gtgctgatta tcagagccat cctcagaata ccctcagtct ctaagaaata caaagccttc   720
   tccacctgtg cctcccattt gggtgcagtc tccctcttct atgggacact ttgtatggta   780
   tacctaaagc ccctccatac ctactctgtg aaggactcag tagccacagt gatgtatgct   840
   gtggtgacac ccatgatgaa tcccttcatc tacagcctga ggaacaagga catgcatggg   900
   gctctgggaa gactcctaga taaacacttt aagaggctga catgagggca atttggaaag   960
   acagcattaa agtggagact at                                            982
<210> 4
   <211> 312
   <212> PRT
   <213> Homo sapiens
<400> 4
   Met Asp Gly Gly Asn Gln Ser Glu Gly Ser Glu Phe Leu Leu Leu Gly
   1 5 10 15
   Met Ser Glu Ser Pro Glu Gln Gln Gln Ile Leu Phe Trp Met Phe Leu
   20 25 30
   Ser Met Tyr Leu Val Thr Val Val Gly Asn Val Leu Ile Ile Leu Ala
   35 40 45
   Ile Ser Ser Asp Ser Arg Leu His Thr Pro Val Tyr Phe Phe Leu Ala
   50 55 60
   Asn Leu Ser Phe Thr Asp Leu Phe Phe Val Thr Asn Thr Ile Pro Lys
   65 70 75 80
   Met Leu Val Asn Leu Gln Ser His Asn Lys Ala Ile Ser Tyr Ala Gly
   85 90 95
   Cys Leu Thr Gln Leu Tyr Phe Leu Val Ser Leu Val Ala Leu Asp Asn
   100 105 110
   Leu Ile Leu Ala Val Met Ala Tyr Asp Arg Tyr Val Ala Ile Cys Cys
   115 120 125
   Pro Leu His Tyr Thr Thr Ala Met Ser Pro Lys Leu Cys Ile Leu Leu
   130 135 140
   Leu Ser Leu Cys Trp Val Leu Ser Val Leu Tyr Gly Leu Ile His Thr
   145 150 155 160
   Leu Leu Met Thr Arg Val Thr Phe Cys Gly Ser Arg Lys Ile His Tyr
   165 170 175
   Ile Phe Cys Glu Met Tyr Val Leu Leu Arg Met Ala Cys Ser Asn Ile
   180 185 190
   Gln Ile Asn His Thr Val Leu Ile Ala Thr Gly Cys Phe Ile Phe Leu
   195 200 205
   Ile Pro Phe Gly Phe Val Ile Ile Ser Tyr Val Leu Ile Ile Arg Ala
   210 215 220
   Ile Leu Arg Ile Pro Ser Val Ser Lys Lys Tyr Lys Ala Phe Ser Thr
   225 230 235 240
   Cys Ala Ser His Leu Gly Ala Val Ser Leu Phe Tyr Gly Thr Leu Cys
   245 250 255
   Met Val Tyr Leu Lys Pro Leu His Thr Tyr Ser Val Lys Asp Ser Val
   260 265 270
   Ala Thr Val Met Tyr Ala Val Val Thr Pro Met Met Asn Pro Phe Ile
   275 280 285
   Tyr Ser Leu Arg Asn Lys Asp Met His Gly Ala Leu Gly Arg Leu Leu
   290 295 300
   Asp Lys His Phe Lys Arg Leu Thr
   305 310
<210> 5
   <211> 24
   <212> DNA
   <213> Artificial Sequence
<220>
   <223> Primer pOR17-4 upstream-2
<400> 5
   actgacctct tctttgtcac caac 24
<210> 6
   <211> 24
   <212> DNA
   <213> Artificial Sequence
<220>
   <223> Primer pOR17-4 downstream-2
<400> 6
   tacatcactg tggctactga gtcc 24
<210> 7
   <211> 626
   <212> DNA
   <213> Artificial Sequence
<220>
   <223> hOR17-4 fragment, transmembrane-domains 3-6
<400> 7
   actgacctct tctttgtcac caacacaatc cccaagatgc tggtgaacct ccagtcccat     60
   aacaaagcca tctcctatgc agggtgtctg acgcagctct acttcctggt ctccttggtg    120
   gccctggaca acctcatcct ggctgtgatg gcatatgacc gctatgtggc catctgctgc    180
   cccctccact acaccacagc catgagccct aagctctgta tcttactcct ttccttgtgt    240
   tgggtcctat ccgtcctcta tggcctcata cacaccctcc tcatgaccag agtgaccttc    300
   tgtgggtcac gaaaaatcca ctacatcttc tgtgagatgt atgtattgct gaggatggca    360
   tgttccaaca ttcagattaa tcacacagtg ctgattgcca caggctgctt catcttcctc    420
   attccctttg gattcgtgat catttcctat gtgctgatta tcagagccat cctcagaata    480
   ccctcagtct ctaagaaata caaagccttc tccacctgtg cctcccattt gggtgcagtc    540
   tccctcttct atgggacact ttgtatggta tacctaaagc cctccatacc ctactctgtg    600
   aaggactcag tagccacagt gatgta                                         626
<210> 8
   <211> 350
   <212> PRT
   <213> Artificial Sequence
<220>
   <223> hOR17-4 fusion protein with hOR17-40
<400> 8
<210> 9
   <211> 22
   <212> DNA
   <213> Artificial Sequence
<220>
   <223> Primer hOR17-40 5' upstream
<400> 9
   aattaatacg actcactata gg 22
<210> 10
   <211> 19
   <212> DNA
   <213> Artificial Sequence
<220>
   <223> Primer hOR17-40 5' downstream
<400> 10
   tactgatagg ttccccagg 19
<210> 11
   <211> 20
   <212> DNA
   <213> Artificial Sequence
<220>
   <223> Primer hOR17-40 3' upstream
<400> 11
   tactgtcatc aatcccatgc 20
<210> 12
   <211> 19
   <212> DNA
   <213> Artificial Sequence
<220>
   <223> Primer hOR17-40 3' downstream
<400> 12
   gatttaggtg acactatag 19

## Beschreibung

Die Erfindung betrifft das Gebiet der olfaktorischen Rezeptoren, der empfängnisbeeinflussenden Medikamente und der Biosensoren.

Seit der Entdeckung der für olfaktorische Rezeptoren (Duftstoffrezeptoren) codierenden DNA-Sequenzen (Gene) sind zahlreiche Aspekte im Zusammenhang mit diesen Rezeptoren noch immer ungeklärt. Zwar kann der Fachmann mit einiger Sicherheit anhand charakteristischer Merkmale - beispielsweise Zugehörigkeit zur Familie der G-Protein gekoppelten Rezeptoren, Hypervariabilität in den Transmembran-Domänen III, IV, V und VI - zwischen DNA-Sequenzen von Duftstoffrezeptoren und solchen Sequenzen, die nicht für einen Duftstoffrezeptor codieren, unterscheiden. Damit ist jedoch noch nichts substantiell über die Regulation der Duftstoffrezeptor-Expression bekannt; insbesondere ist unbekannt, in welchem Gewebe und in welcher Stärke ein vermutetes Duftstoffrezeptor-Gen exprimiert wird; ferner ist nicht vorhersagbar, welche Substanzen an den von diesem Gen codierten Duftstoffrezeptor spezifisch binden können, sei es als Agonist (Aktivator) oder als Antagonist (Inhibitor).

Bisher ist erst bei zwei menschlichen Duftstoffrezeptor-Genen (hOR17-40, s. Wetzel et al., The Journal of Neuroscience, 1999: 7426-7433 und hOR17-4, s. Nekrasova et al., European Journal of Biochemistry, 1996, 28-37) die funktionelle Expression des zugehörigen Rezeptor-Proteins gelungen. Es konnten nur zwei als Agonisten wirkende Substanzen bestimmt werden, nämlich Helional und Heliotropylaceton. Diese Substanzen lösen eine Aktivierung des Rezeptors aus, die sich in einer vorübergehenden Erhöhung des cytosolischen Ca²⁺-Spiegels niederschlägt, wenn der Rezeptor funktionell in HEK293-Zellen exprimiert wird. Antagonisten, also Substanzen, die eine Rezeptor-Aktivierung durch einen oder mehrere Agonisten spezifisch und reversibel verhindern können, wurden nicht gefunden. Der olfaktorische Rezeptor OR5 aus Ratten wurde in *E. coli* exprimiert und seine spezifische Bindung an Lilial nachgewiesen (Kiefer et al., Biochemistry, 1996, 16077-16084). Darüberhinaus ist es nach wie vor nicht möglich, anhand der DNA-Sequenz bzw. der Rezeptor-Aminosäuresequenz Vorhersagen darüber zu treffen, welche Substanzen als Agonisten oder Antagonisten wirken können.

Es besteht daher ein großer Bedarf, weitere Duftstoffrezeptoren zu finden, funktionell zu exprimieren und die jeweils wirksamen Agonisten und Antagonisten zu bestimmen.

Es wird daher gemäß einem ersten Aspekt der Erfindung ein Rezeptor angegeben, der
a) einen Proteinabschnitt mit einer Aminosäuresequenz gemäß SEQ ID NO. 8 umfasst.

Im Zusammenhang mit der vorliegenden Erfindung werden zudem Rezeptoren beschrieben, die
b) einen Proteinabschnitt mit einer Aminosäure-Sequenzhomologie von mehr als 70% zu einem Proteinabschnitt gemäß Alternative a), oder
c) ein Fragment eines Proteinabschnitts gemäß einer der Alternativen a) oder b) umfassen.

Es ist nunmehr erstmals gelungen, ein Fusionsprotein der Aminosäuresequenz SEQ ID NO. 8 funktionell zu exprimieren. Ausgangspunkt hierbei war die unter der Bezeichnung OR17-4 (wegen der Nomenklatur siehe Ben-Arie et al., Human Molecular Genetics (1994), 229-235) veröffentlichte Nucleinsäuresequenz des für den Rezeptor codierenden Gens (Gensequenz) eines menschlichen Duftstoffrezeptors. Eine synonyme Bezeichnung ist OR1 D2, unter dieser Bezeichnung ist die Gensequenz in der GenBank-Datenbank veröffentlicht (GenBank ACC. number NM_002548, EMBL-accession number AF087917). Der Rezeptor gehört zu der eingangs beschriebenen Klasse von Proteinen, die zur Superklasse der G-Proteingekoppelten Proteine mit sieben vermuteten Transmembrandomänen gehören. Da nunmehr die funktionelle Expression dieses Rezeptors als Fusionsprotein mit der Aminosäuresequenz SEQ ID NO. 8 erstmals gelungen ist, wurde es auch möglich, Substanzen zu finden, die als Agonist oder Antagonist spezifisch an den Rezeptor binden können; hierzu unten mehr.

Die Aminosäuresequenz SEQ ID NO. 8 betrifft ein Fusionsprotein, das die vermuteten Transmembran-Domänen III, IV, V und VI sowie hieran angrenzend Abschnitte der vermuteten Transmembrandomänen II und VII des Rezeptors der Aminosäuresequenz SEQ ID NO. 4 enthält. Es hat sich gezeigt, dass dieser Abschnitt, insbesondere wenn er zusammen mit den vermuteten Transmembran-domänen I und VII des Rezeptors hOR17-40 in Form eines Fusionsproteins exprimiert wird, es einem Protein ermöglicht, an Agonisten und an den Antagonisten aus derjenigen Gruppe von Substanzen spezifisch zu binden, an die auch das Protein mit der Aminosäuresequenz gemäß SEQ ID NO. 4 spezifisch bindet.

Der Fachmann erkennt, dass auch ein Protein, das einen Proteinabschnitt mit einer von der Sequenz SEQ ID NO. 8 abweichenden Aminosäuresequenz besitzt, ein Rezeptor mit erfindungsgemäßer Funktion sein kann. Weicht der Proteinabschnitt von der Sequenz SEQ ID NO. 8 ab, so ist es bevorzugt, wenn er zu der Sequenz homolog ist. Die Erfolgsaussichten, einen funktionalen Rezeptor zu erhalten, insbesondere einen Duftstoffrezeptor, ist in der Gruppe der Proteine mit einem Abschnitt mit homologer Aminosäuresequenz zu der Sequenz SEQ ID NO. 8 höher als in der Gruppe der Proteine ohne oder mit nur geringer Homologie zu der genannten Sequenz. Beschrieben werden daher im Zusammenhang mit der vorliegenden Erfindung auch solche Proteine, die einen Proteinabschnitt mit einer Aminosäure-Sequenzhomologie von mehr als 70 % zu einem Proteinabschnitt gemäß SEQ ID NO. 8 umfassen. Die Aminosäure-Sequenzhomologie kann zweckmäßigerweise mit Hilfe des EMBOSS:water-Programms (Algorithmus von Smith und Waterman (1981), Identification of common molecular subsequences, J. Mol. Biol. 147:195-197) berechnet werden (Gap open penalty: 10.0; Gap extension penalty: 0.5; Blosum62-Matrix). Das Programm berechnet einen "Similarity"-Prozentwert, dieser ist das Maß der Homologie. Ein "Similarity"-Prozentwert von 80% bedeutet also im Sinne dieser Erfindung eine Sequenzhomologie von 80%. Beispielsweise haben die Sequenzen SEQ ID NO. 4 und SEQ ID NO. 8 zueinander eine "Similarity" und daher eine Homologie von 90,7%.

Ferner werden im Zusammenhang mit der vorliegenden Erfindung Rezeptoren beschrieben, die ein Fragment eines Proteinabschnitts gemäß der Sequenz SEQ ID NO. 8 oder eines zu einem solchen Proteinabschnitt homologen, insbesondere zu mehr als 70 % homologen, Proteinabschnitts umfassen. Der Fachmann wird dabei, insbesondere wenn er an einem funktionalen Duftstoffrezeptor interessiert ist, zweckmäßigerweise solche Proteine auswählen, die einen oder mehrere derjenigen Agonisten und/oder Antagonisten binden, an die ein Protein mit einem Abschnitt gemäß Alternative a) bzw. b) bindet. Insbesondere kann es, wenn neben dem Fragment gemäß Alternative c) weitere Proteinabschnitte in dem auszuwählenden Rezeptor vorhanden sind, ausreichend sein, wenn das Fragment gemäß Alternative c) nur eine oder mehrere Teilsequenzen eines Proteinabschnitts gemäß Alternative a) oder b) umfasst.

Es steht zu vermuten, dass der Rezeptor gemäß SEQ ID NO. 4 ein Bindungszentrum besitzt, das für die spezifische Bindung an einen oder mehrere Agonisten verantwortlich ist. Insbesondere kann vermutet werden, dass dieses Bindungszentrum von den Transmembrandomänen III, IV, V und VI gebildet wird. Es kann also ausreichend sein, wenn das Fragment gemäß Alternative c) diejenigen Aminosäuren bzw. deren Seitengruppen umfasst, die für die spezifische Bindung im aktiven Zentrum des Rezeptors gemäß Alternative a) verantwortlich sind, wobei es insbesondere zweckmäßig sein kann, diese Aminosäuren im Fragment gemäß Alternative c) in der gleichen räumlichen Anordnung vorzusehen, wie sie auch in einem Rezeptor gemäß Alternative a) vorliegen.

Wird der Rezeptor als heterologes Protein in einer Wirtszelle exprimiert - auch hierzu unten mehr-, so ist es möglich, dass die Transkription und/oder Translation bestimmter Nucleinsäure-Codons bzw. Codon-Abfolgen effizienter vonstatten geht als die Transkription und/oder Translation anderer Codons bzw. Codon-Abfolgen. Hieraus kann sich eine Präferenz für bestimmte Aminosäuren und/oder Aminosäureabfolgen ergeben. Es ist nunmehr bevorzugt, wenn einige oder alle Aminosäuren des Rezeptors, für die keine Präferenz der Wirtszelle besteht, durch solche Aminosäuren ersetzt sind, für die eine Präferenz der Wirtszelle besteht. Ferner kann es vorteilhaft sein, eine Protease-Schnittstelle durch einen Aminosäureaustausch, -deletion oder -insertion zu entfernen.

Vorzugsweise ist der Rezeptor ein Fusionsprotein, das neben einem Proteinabschnitt gemäß den Alternativen a) (erfindungsgemäß) oder b) oder einem Fragment gemäß Alternative c) einen oder mehrere weitere Proteinabschnitte umfasst. Hierbei kann es sich insbesondere um Signalpeptide wie die "5HT₃-Sequenz" (vergleiche C. H. Wetzel et al., Journal of Neuroscience 19, 7426-7433 (1999)) handeln, die den Transport und den funktionellen Einbau in die Zellmembran erleichtert. Anstelle oder neben der "5HT₃-Sequenz" können jedoch auch andere Signalpeptide verwendet werden, insbesondere solche, die einen intra- und/oder interzellulären Transport des Fusionsproteins steuern oder erleichtern.

Im Zusammenhang mit der vorliegenden Erfindung wird - als zweiter Aspekt (nicht erfindungsgemäß) - auch ein Rezeptor beschrieben, der strukturell geeignet beschaffen ist, spezifisch an einen Agonisten zu binden, der
- eine Aldehydgruppe umfasst, deren Kohlenstoffatom
- über eine 1, 2 oder 3 Atome lange Kette
- mit einem aromatischen Rest verbunden ist, wobei
- die Kette maximal ein Sauerstoffatom umfassen darf und ansonsten aus Kohlenstoffatomen besteht,
- jedes Kohlenstoffatom der Kette unabhängig von jedem anderen dieser Kohlenstoffatome mit einem oder zwei Methylresten verbunden sein darf, und
- der aromatische Rest in ortho-, meta- oder para-Stellung zur Kette mit -CH₃, -C₂H₅, -O-CH₃, -CH₂-CH₃, -CH(CH₃)₂ oder -C(CH₃)₃ substituiert sein kann.

Ein Beispiel eines solchen Rezeptors ist der Rezeptor mit der Aminosäuresequenz SEQ ID NO. 4.

Unter einer spezifischen Bindung im Sinne dieser Erfindung wird eine Bindung verstanden, die - vereinfacht gesagt - nach einer Art Schlüssel-Schloss-Prinzip funktioniert. Insbesondere bindet der Rezeptor eine Substanz bzw. bindet eine Substanz an den Rezeptor dann spezifisch, wenn die Substanz bei Applikation auf mehrere Moleküle des Rezeptors bei niedrigen Konzentrationen, vorzugsweise bei Konzentrationen kleiner als 10 mM, besonders bevorzugt bei Konzentrationen kleiner oder gleich 1 mM, insbesondere bevorzugt bei Konzentrationen kleiner oder gleich 100 µM, mit großer Wahrscheinlichkeit immer wieder im gleichen Bereich des Rezeptors bindet. Das Ergebnis einer solchen spezifischen Bindung kann insbesondere eine - vorzugsweise reversible - nicht im wesentlichen lediglich auf den Ort der Bindung begrenzte Konformationsänderung des Rezeptors sein.

Ferner wird unter einem Agonisten im Sinne der Erfindung eine Substanz verstanden, die durch ihre spezifische Bindung an den Rezeptor eine über diese bloße Bindung hinausgehende chemische Reaktion auslöst. In ihrem natürlichen Umfeld stehen Rezeptoren nicht chemisch unvermittelt neben anderen Zellbestandteilen, sondern stehen in Wirkverbindung mit weiteren Teilen zumindest einer Signaltransduktionskaskade, wie sie beispielsweise durch G-Proteine vermittelt wird. Durch das spezifische Binden eines Agonisten werden solche Rezeptoren von einem inaktiven in einen aktiven Zustand überführt, dies geht gewöhnlich einher mit einer nicht im wesentlichen auf den Ort der Agonisten-Bindung begrenzten Konformationsänderung des Rezeptors und schlägt sich in einem Einschalten der Signaltransduktionskaskade nieder, erkennbar beispielsweise an einem Anstieg der cytosolischen Konzentration von Ca²⁺, cAMP, cGMP und/oder Inositol-Triphosphat oder der Änderung des ATP/ADP-Quotienten. Die Konformationsänderung des Rezeptors, und auch das Einschalten der Signaltransduktionskaskade, ist eine über die bloße Bindung des Agonisten hinausgehende chemische Reaktion.

Besonders bevorzugt ist dabei ein Rezeptor, der strukturell geeignet beschaffen ist, spezifisch an einen Agonisten der Formel I 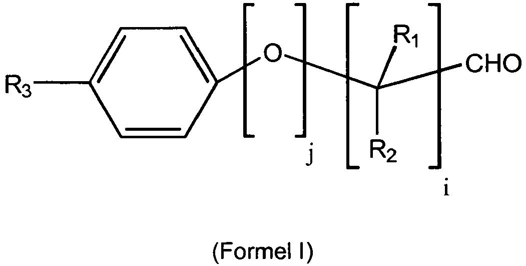 zu binden, wobei
i: 1, 2 oder 3 ist,
j: 0 oder 1 ist,
jeder der Reste R1, R2 unabhängig von jedem anderen gegebenenfalls vorhandenen Rest R1, R2 -H oder -CH₃ ist und
R3 -H, -CH₃, -C₂H₅, -O-CH₃, -CH₂-CH₃, -CH(CH₃)₂ oder -C(CH₃)₃ ist.

Insbesondere sind solche Rezeptoren bevorzugt, bei denen die Summe von i + j = 1, 2 oder 3 ist. Es hat sich gezeigt, dass ein Rezeptor mit einer Aminosäuresequenz gemäß SEQ ID NO. 4 und ein Fusionsprotein mit der Aminosäuresequenz SEQ ID NO. 8 an solche Substanzen besonders gut spezifisch bindet, siehe hierzu auch die unten folgenden Beispiele.

Besonders bevorzugt ist dabei ein Rezeptor, der strukturell geeignet beschaffen ist, spezifisch an einen Agonisten zu binden, der ausgewählt ist aus der Gruppe bestehend aus 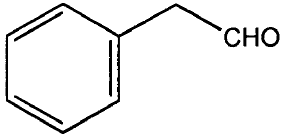 (Phenylacetaldehyd, CAS-Nummer 122-78-1), 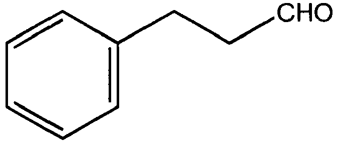 (3-Phenylpropanal, CAS-Nummer 104-53-0), 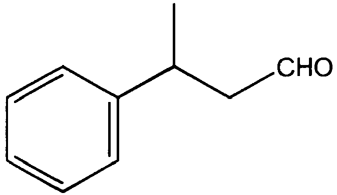 (3-Phenylbutyraldehyd, CAS-Nummer 16251-77-7), 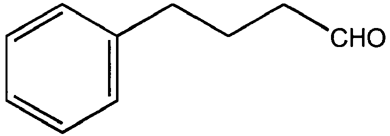 (4-Phenylbutyraldehyd, CAS-Nummer 18328-11-5), 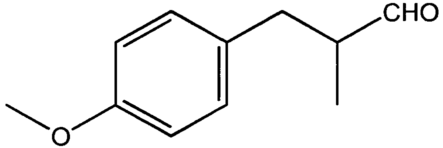 (Canthoxal, CAS-Nummer 5462-06-6), 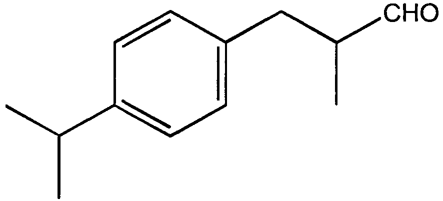 (Cyclamal, CAS-Nummer 103-95-7), 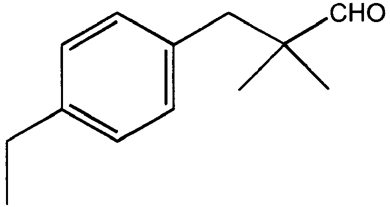 (Floralazon, CAS-Nummer 67634-15-5), 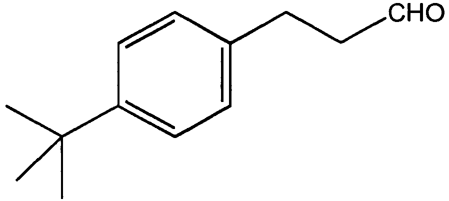 (Bourgeonal, CAS-Nummer 18127-01-0), 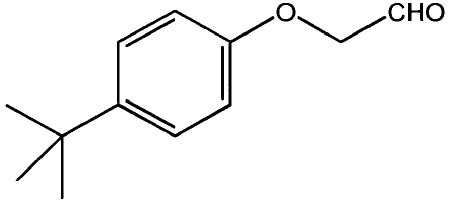 ((4-tert-butylphenoxy)acetaldehyd, CAS-Nummer 72928-50-8).

Besonders bevorzugt ist es ferner, wenn der Rezeptor ein G-Protein gekoppelter Rezeptor ist. Solche Rezeptoren sehen am Anfang zahlreicher für das sensorische Empfinden, insbesondere für die Duftstoffwahrnehmung des Menschen, maßgeblicher Signaltransduktionskaskaden.

Erfindungsgemäß besonders bevorzugt sind solche Rezeptoren, die sowohl Rezeptoren gemäß dem ersten (erfindungsgemäßen) als auch gemäß dem zweiten (hierin im Zusammenhang mit der vorliegenden Erfindung beschriebenen) Aspekt der Erfindung sind. Von diesen Rezeptoren ist durch die Erfindung sowohl deren grundlegende Aminosäuresequenz wie eingangs beschrieben, insbesondere die gemäß SEQ ID NO. 4, bekannt, als auch können - wie beschrieben - eine Reihe von Agonisten angegeben werden, die an diesen Rezeptoren spezifisch binden.

Weiterhin wird hierin im Zusammenhang mit der vorliegenden Erfindung - als dritter Aspekt (nicht erfindungsgemäß) - ein Rezeptor beschrieben, der strukturell geeignet beschaffen ist, spezifisch an n-Undecanal zu binden. Diese Substanz gehört nicht zu den Agonisten gemäß Formel I. Sie erweitert jedoch das Spektrum an Substanzen, deren spezifisches Binden an den Rezeptor nachweisbar ist.

Vorzugsweise ist der erfindungsgemäße Rezeptor ein Rezeptor gleichzeitig gemäß allen dreien Aspekten (wie oben beschrieben). Mit den Rezeptoren gemäß dem ersten Aspekt der Erfindung konnte nunmehr erstmals eine Klasse von Rezeptoren angegeben werden, zu der Rezeptoren gehören, die sowohl die zuvor aufgezählten Agonisten als auch n-Undecanal spezifisch binden können.

Dabei sind jene Rezeptoren besonders bevorzugt, die strukturell so beschaffen sind, dass n-Undecanal als Antagonist gegen das spezifische Binden eines Agonisten der Formel I, vorzugsweise eines Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd, an den Rezeptor wirkt.

Im Sinne der Erfindung ist ein Antagonist eine Substanz, die zwar spezifisch von einem Rezeptor gebunden wird, jedoch das spezifische Binden eines Agonisten an den Rezeptor erschwert. Das spezifische Binden des Antagonisten führt, anders als bei einem Agonisten, nicht zum Einschalten einer Signaltransduktionskaskade, wenn der Rezeptor Teil einer solchen, durch das spezifische Binden eines Agonisten eingeschalteten Signaltransduktionskaskade ist. Insbesondere kann eine durch das spezifische Binden eines Agonisten hervorgerufene Konformationsänderung des Rezeptors beim spezifischen Binden eines Antagonisten ausbleiben.

Gemäß einem weiteren Aspekt der Erfindung wird ein Vektor angegeben, der einen Nucleinsäure-Abschnitt umfasst, der für einen Rezeptor nach dem ersten Aspekt der Erfindung codiert. Ein solcher Vektor erleichtert es, eine für den erfindungsgemäßen Rezeptor codierende Nucleinsäure zu klonieren. Der Vektor kann auch ein sogenannter suicide-Vektor sein, insbesondere ein solcher, der die Integration des für den Rezeptor codierenden Nucleinsäure-Abschnitts in das Genom der Wirtszelle ermöglicht.

In besonders bevorzugten Ausführungsformen ist der Vektor ein Expressionsvektor. Solche Vektoren ermöglichen die Transkription und Translation des im Vektor enthaltenen, für den erfindungsgemäßen Rezeptor codierenden Nucleinsäureabschnitts, wenn der Vektor in eine geeignete Wirtszelle eingebracht wird. Geeignete Wirtszellen sind insbesondere solche aus *Drosophila melanogaster, Caenorhabditis elegans, Xenopus laevis*-Oocyten und menschliche HEK293-Zellen sowie CHO-(Chinese Hamster Ovary), COS- (Afrikan Green Monkey Kidney), BHK- (Syrian Baby Hamster Kidney) und PC12- (Rat Adrenal Phenochromocytoma) - Zellen.

Gemäß einem weiteren Aspekt der Erfindung wird ein Expressionssystem angegeben, das eine Zelle umfasst, die einen erfindungsgemäßen Rezeptor überexprimiert. Unter "überexprimieren" wird dabei jeder Vorgang verstanden, in dessen Folge der Rezeptor über das für die jeweilige Wirtszelle unter natürlichen Bedingungen maximal erreichbare Genexpressionsniveau hinausgehend vorliegt. Dabei kann der Rezeptor insbesondere auch ein für die Wirtszelle heterologer Rezeptor sein. Stellt die betreffende Wirtszelle normalerweise keinen erfindungsgemäßen Rezeptor her, so findet eine Überexpression im Sinne der Erfindung bereits dann statt, wenn sie (erstmals) überhaupt einen solchen Rezeptor herstellt. Besonders bevorzugt sind dabei solche Wirtszellen, die neben der funktionellen Expression des Rezeptors auch die für eine vom Rezeptor durch die Bindung eines Agonisten beeinflusste Signaltransduktionskaskade notwendigen Elemente bereitstellen.

Zweckmäßigerweise handelt es sich bei der Wirtszelle um eine Säugerzelle, wobei die Wirtszelle insbesondere auch eine entartete Säugerzelle sein kann. Ein Beispiel hierfür ist die auf humane embryonale Nierenzellen zurückgehende Zellinie HEK293 (s. Graham *et al.,* J. Gen. Virol. (1977): 59-72).

Gemäß einem weiteren (nicht erfindungsgemäßen) Aspekt im Zusammenhang mit der vorliegenden Erfindung wird ein Komplex angegeben, wobei der Komplex einen erfindungsgemäßen Rezeptor, sowie daran spezifisch gebunden,
a) einen Agonisten der Formel I, vorzugsweise einen Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd, oder
b) einen Antagonisten gegen das spezifische Binden eines Agonisten nach a), vorzugsweise n-Undecanal
umfasst.

Ein solcher Komplex ist besonders vorteilhaft dazu geeignet, die Wechselwirkungen zwischen dem Rezeptor und einem Agonisten und/oder einem Antagonisten zu untersuchen. Dabei kann es sich bei dem Antagonisten um einen reversiblen oder irreversiblen handeln. Wenn der Rezeptor mit einer Signaltransduktionskaskade in Wirkverbindung steht, so ermöglicht das Herstellen eines solchen Komplexes aus Rezeptor und Agonist das Einschalten oder Aktivieren der entsprechenden Signaltransduktionskaskade. Das Herstellen eines Rezeptor-Antagonisten-Komplexes ermöglicht es in diesem Fall, das Einschalten der Signaltransduktionskaskade auch bei Vorliegen des Agonisten zu verhindern. Weitere vorteilhafte Anwendungsmöglichkeiten eines Komplexes aus Rezeptor und Agonist oder Antagonist sind unten beschrieben. Besonders bevorzugt sind dabei die Komplexe aus einem erfindungsgemäßen Rezeptor und Bourgeonal oder n-Undecanal. Beide bilden sich schon bei geringen Konzentrationen an Agonist bzw. Antagonist.

Insbesondere wird gemäß einem weiteren Aspekt der Erfindung ein Biosensor angegeben, der
a) einen erfindungsgemäßen Rezeptor mit einem Proteinabschnitt mit einer Aminosäuresequenz gemäß SEQ ID NO. 8, und
b) Mittel zum Nachweisen einer Bindung eines Agonisten an den Rezeptor, wobei der Agonist ausgewählt ist aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd,
umfasst.

Der Fachmann kann die zum Nachweisen der Bindung des Agonisten an den Rezeptor geeigneten Mittel je nach der Art des gewählten Rezeptors leicht auswählen. In einer bevorzugten Ausführungsform umfasst der Biosensor eine Zelle, die einen erfindungsgemäßen Rezeptor exprimiert, und in der der Rezeptor in Wirkverbindung mit einer Signaltransduktionskaskade steht. Durch das spezifische Binden des Agonisten an den Rezeptor wird ein Rezeptor-Agonist-Komplex wie soeben beschrieben hergestellt. Dabei kann es zu einer nicht im wesentlichen lediglich auf den Ort der Bindung des Agonisten begrenzten Konformationsänderung des Rezeptors kommen. Durch diese Konformationsänderung oder auf andere Weise löst der Rezeptor nach der Bindung des Agonisten die Signaltransduktionskaskade aus. Diese wiederum erzeugt ein messbares Signal, beispielsweise in Form einer Erhöhung der cytosolischen Ca²⁺-Konzentration. Anstelle der cytosolischen Ca²⁺ -Konzentration kann auch der Konzentrationsanstieg oder der Konzentrationsabfall einer anderen Substanz, insbesondere eines second messegers wie cAMP, cGMP, IP₃ und dergleichen gemessen werden. Der Fachmann kann anhand der vom Rezeptor durch die spezifische Bindung eines Agonisten beeinflusste Signaltransduktionskaskade leicht eine geeignete Substanz zum Nachweisen dieser Bindung auswählen, deren Konzentration oder Konformation sich entsprechend der spezifischen Bindung des Agonisten an den Rezeptor ändert.

Ein solcher Biosensor ist insbesondere vorteilhaft dazu geeignet, weitere Rezeptoren zu suchen. Dazu wird der Fachmann zunächst ein Expressionssystem bereitstellen, in dem der vermutete Rezeptor funktionell exprimiert wird. Auf das Expressionssystem werden vermutete Agonisten appliziert und eine gegebenenfalls hierdurch ausgelöstes Signal des Biosensors gemessen. Wenn eine Substanz ein Biosensor-Signal auslöst und dieses Signal nicht auch in den natürlichen Zellen des Expressionssystems (also Zellen, die nicht zu einem Expressionssystem verändert wurden) auftritt, so wird vom Expressionssystem tatsächlich ein Rezeptor für diese Substanz exprimiert.

Ebenfalls wird im Zusammenhang mit der vorliegenden Erfindung eine Sonde zum Nachweisen einer Nucleinsäure beschrieben, die für einen Abschnitt eines erfindungsgemäßen Rezeptors codiert. Eine solche Sonde ist zweckmäßigerweise eine Nucleinsäure, insbesondere eine RNA oder eine, vorzugsweise einzelsträngige, DNA, wobei die Nucleinsäure mit einer Nucleinsäure hybridisieren kann, die für einen Abschnitt eines erfindungsgemäßen Rezeptors codiert (z.B. einer Nucleinsäure gemäß SEQ ID NO. 3). Die Nucleinsäure ist verbunden mit einer nachweisbaren Markierung, beispielsweise mit einer Fluoreszenz-, Lumineszenz-, Farbmarkierung, einer radioaktiven Markierung oder einem Enzym, das die Bildung eines nachweisbaren Reaktionsprodukts katalysiert. Anstelle einer solchen nachweisbaren Markierung kann die Sonde auch an einen Feststoff, beispielsweise an Metallpartikel wie magnetische Beads, gekoppelt sein (gegebenenfalls über einen Linker).

In diesem Zusammenhang wird gelehrt, eine Nucleinsäure, vorzugsweise eine Sonde wie zuvor beschrieben, zum Nachweisen einer Nucleinsäure, die für ein Fragment eines Rezeptors codiert, zu verwenden. So können zum einen weitere Rezeptoren mit gleicher oder ähnlicher Aminosäuresequenz wie der erfindungsgemäße Rezeptor gefunden werden. Dabei wird die Sonde unter vorzugsweise stringenten Hybridisierungsbedingungen mit Nucleinsäuren einer zu untersuchenden Zelle in Kontakt gebracht, um ein Hybridisieren der Sonde an eine dieser entsprechenden Wirts-Nucleinsäure zu ermöglichen, falls eine solche entsprechende Nucleinsäure vorliegt. Je weniger stringent die Hybridisierungsbedingnungen sind, desto weniger ähnlich können die Nucleinsäuren sein, die mit der Sonde hybridisieren. Nach dem Hybridisieren werden die mit der Sonde hybridisierten Nucleinsäuren aufgereinigt und sequenziert.

Soll mit der Sonde nur die Anwesenheit von Nucleinsäuren, die für ein Fragment eines Rezeptors codieren, nachgewiesen werden, so reicht es aus, diese Nucleinsäuren - vorzugsweise unter stringenten Hybridisierungsbedingungen - mit der Sonde in Kontakt zu bringen, um ein Hybridisieren der Sonde an die entsprechende Nucleinsäure zu ermöglichen, wenn eine solche entsprechende Nucleinsäure vorliegt. Dabei ist es zweckmäßig, wenn die Nucleinsäure, die mit der Sonde nachgewiesen werden soll, an einem festen Träger immobilisiert ist, beispielsweise in einem Gel oder auf einem Nylonfilter. In diesem Fall ist das Hybridisieren der Sonde an die nachzuweisende Nucleinsäure leicht dadurch nachweisbar, dass die Sonde - vermittelt durch die nachzuweisende Nucleinsäure - ebenfalls an den festen Träger gebunden ist und bei Durchführen eines Waschschrittes im wesentlichen nicht vom festen Träger abgewaschen wird. Dem Fachmann sind derartige Nachweisverfahren als Southern- oder Northern-Blot bekannt.

Beispielsweise kann eine Nucleinsäure gemäß einer der Sequenzen SEQ ID NO. 1, SEQ ID NO. 2, SEQ ID NO. 5 oder SEQ ID NO. 6 zum Amplifizieren einer Nucleinsäure, die für einen Rezeptor nach Anspruch 1 oder einen Abschnitt eines solchen Rezeptors codiert, verwendet werden. Insbesondere können die Nucleinsäuren gemäß den Sequenzen SEQ ID NO. 1 und SEQ ID NO. 2 als Primer für eine Polymerase-Kettenreaktion (PCR) zur Amplifikation einer für den Rezeptor gemäß SEQ ID NO. 4 codierenden Nucleinsäure verwendet werden, wobei die Sequenzen SEQ ID NO. 1 und SEQ ID NO. 2 vorzugsweise gemeinsam als Primerpaar verwendet werden. Die Nucleinsäuren gemäß SEQ ID NO. 5 und SEQ ID NO. 6 können insbesondere gemeinsam als Primerpaar zum Amplifizieren eines solchen Abschnitts des Rezeptors gemäß SEQ ID NO. 4 dienen, der die vermuteten Transmembran-Domänen III - VI umfasst. Die amplifizierten Nucleinsäuren können auch, insbesondere nach Anbringen einer entsprechenden Markierung, als Sonde wie oben beschrieben verwendet werden. Darüber hinaus können sie auch ohne Verwendung einer Sonde den Nachweis ermöglichen, dass in einem Amplifikationsansatz eine für einen erfindungsgemäßen Rezeptor codierende Nucleinsäure vorhanden ist, vorausgesetzt, es wird eine Nucleinsäure amplifiziert, deren Größe charakteristisch für ein Amplifikationsprodukt einer für einen erfindungsgemäßen Rezeptor codierenden Nucleinsäure ist.

Gemäß einem weiteren Aspekt im Zusammenhang mit der vorliegenden Erfindung wird ein Agonist der Formel I, vorzugsweise ein aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd ausgewählter Agonist, zum spezifischen Binden an einen erfindungsgemäßen Rezeptor verwendet. Mit dieser Verwendung ergeben sich die oben für den erfindungsgemäßen Rezeptor beschriebenen Vorteile. Insbesondere ist es möglich, dass durch das spezifische Binden eines Agonisten eine chemische Reaktion asugelöst wird. Wenn der Rezeptor in Wirkverbindung mit einer Signaltransduktionskaskade steht, kann insbesondere durch das Binden des Agonisten diese Signaltransduktionskaskade eingeschaltet werden.

Entsprechend der Verwendung eines Agonisten wird auch die Verwendung einer Substanz, vorzugsweise von n-Undecanal, als Antagonist gegen das spezifische Binden eines Agonisten der Formel I, vorzugsweise eines aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd ausgewählten Agonisten, an einen erfindungsgemäßen Rezeptor angegeben. Mit dieser Verwendung können die oben im Zusammenhang mit dem Antagonisten beschriebenen Vorteile und Effekte erzielt werden.

Ein erfindungsgemäßer Rezeptor kann zum spezifischen Binden
a) eines Agonisten der Formel I, vorzugsweise eines Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd, oder
b) eines Antagonisten gegen das spezifische Binden eines Agonisten gemäß a), vorzugsweise n-Undecanal,
verwendet werden. Dadurch werden die oben beschriebenen Komplexe erzeugt, die die oben genannten Vorteile besitzen.

Eine besonders bevorzugte erfindungsgemäße nicht-therapeutische Verwendung
a) eines Agonisten der Formel I, vorzugsweise einen Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd, und/oder
b) eines Antagonisten gegen das spezifische Binden eines Agonisten gemäß a), vorzugsweise von n-Undecanal,
besteht darin, die Bewegungsrichtung und/oder die Geißelschlagfrequenz eines Spermiums zu beeinflussen. Es hat sich herausgestellt, dass auch Spermien Rezeptoren gemäß dem ersten Aspekt der Erfindung exprimieren und chemotaktische Reaktionen zeigen, die durch die Bindung eines Agonisten gemäß a) ausgelöst werden können. Insbesondere schwimmen Spermien in einem Konzentrationsgradienten eines solchen Agonisten in Richtung auf die höhere Agonisten-Konzentration. Dieser Effekt kann dazu genutzt werden, Spermien eine bestimmte Schwimmrichtung vorzugeben. Beispielsweise ist es auf diese Weise möglich, Spermien in einem vorgewählten Bereich in einer flüssigen Probe anzureichern. Zudem erhöht sich die Geißelschlagfrequenz von Spermien bei Zugabe eines Agonisten gemäß a). Die Agonisten können daher auch dazu verwendet werden, die Schwimmgeschwindigkeit von Spermien zu erhöhen. Die Zugabe eines Antagonisten zu Spermien führt dazu, dass die beschriebenen Effekte - bei gleichbleibender Agonistenkonzentration - entweder nicht oder nicht in dem sonst üblichen Maße eintreten. Damit kann insbesondere in einer flüssigen Probe, in der ein Agonisten-Konzentrationsgradient besteht, eine Anreicherung von Spermien im Bereich höherer Agonisten-Konzentrationen verhindert werden.

Dementsprechend wird erfindungsgemäß auch eine Verwendung als Kontrazeptivum und eine Anwendung als empfängnisförderndes Arzneimittel angegeben, wobei das Kontrazeptivum bzw. das Arzneimittel
a) einen Agonisten der Formel I, vorzugsweise einen Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd, und/oder
b) n-Undecanal,

in einer kontrazeptiven oder empfängnisfördernden Menge umfassen. Unter einem Arzneimittel wird dabei jedes Mittel verstanden, das zur Vorbeugung, Diagnose oder zur Behandlung eines körperlichen Zustandes eines Menschen und/oder Tieres eingesetzt wird. Arzneimittel im erfindungsgemäßen Sinne können daher insbesondere Arzneimittel gemäß § 1 des Arzneimittelgesetzes sein, aber auch Medizinprodukte gemäß § 3 des Medizinproduktegesetzes, jeweils in ihrer am 01. August 2002 geltenden Fassung. Ein solches Arzneimittel nutzt die mit der oben beschriebenen Verwendung von Agonisten und Antagonisten erzielbaren Vorteile. Neben den Agonisten und/oder dem Antagonisten umfasst das Arzneimittel zweckmäßigerweise einen pharmazeutisch akzeptablen Träger. Anstelle des Agonisten oder Antagonisten selbst kann das Arzneimittel auch eine Vorstufe des Agonisten bzw. Antagonisten umfassen, die im menschlichen und/oder tierischen Körper zu einem Agonist bzw. Antagonist umgesetzt wird.

Bei einem kontrazeptiv wirkenden Arzneimitteln kann es besonders zweckmäßig sein, einen Antagonisten - bevorzugt einen den Rezeptor irreversibel hemmenden Antagonisten - im oder auf dem menschlichen Körper, vorzugsweise in der Scheide, dem Uterus und/oder dem Eileiter freizusetzen, um dort gegebenenfalls vorhandenen Spermien die Orientierung hin zu einer gegebenenfalls vorhandenen Eizelle zu erschweren. Dies kann durch ein Intravaginalpräparat erfolgen, aus dem der Antagonist - gegebenenfalls also n-Undecanal - freigesetzt wird. Ebenfalls möglich ist es, ein Präservativ mit einem Antagonisten - wiederum bevorzugt einem irreversiblen - zu versehen, beispielsweise zu imprägnieren oder zu beschichten, so dass der Antagonist bei bestimmungsgemäßer Verwendung des Präservativs freigesetzt wird. Es kann jedoch auch - gegebenenfalls in Kombination mit der Verwendung eines Antagonisten - der Bildung eines positiv chemotaktisch wirkenden Gradienten vorgebeugt werden, indem eine gleichmäßige Konzentration eines Agonisten im Eileiter und/oder Uterus eingestellt wird. Ebenfalls kann es in einem kontrazeptiven Arzneimittel wünschenswert sein, einen von einer Eizelle wegweisenden chemischen Agonisten-Gradienten aufzubauen, um so die Spermien von der Eizelle weg zu lenken. Beides kann insbesondere auch durch entsprechende agonistenhaltige Intravaginalpräparate bzw. Präservative erreicht werden.

Bei einem empfängnisfördernden Arzneimittel kann es insbesondere vorteilhaft sein, einen oder mehrere Agonisten - gegebenenfalls in Kombination mit weiteren Substanzen - im Eileiter und/oder Uterus freizusetzen, um Spermien an den jeweiligen Freisetzungsort zu locken.

Ferner wird erfindungsgemäß n-Undecanal gegen das spezifische Binden eines Agonisten der Formel I, vorzugsweise einen Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd, an einen Rezeptor mit einem Proteinabschnitt mit einer Aminosäuresequenz gemäß SEQ ID NO. 4 oder SEQ ID NO. 8, oder mit einem Proteinabschnintt mit einer Aminosäure-Sequenzhomologie von mehr als 70% zu einem Proteinabschnitt mit einer Aminosäuresequenz gemäß SEQ ID NO. 4 oder SEQ ID NO. 8, zum Beeinflussen der Geruchswahrnehmungsfähigkeit eines Menschen und/oder eines Tieres nicht-therapeutisch verwendet. Die Verwendung eines Antagonisten ermöglicht es auf einfache Weise, die Geruchswahrnehmungsschwelle für einen oder mehrere Agonisten bei Mensch und/oder Tier anzuheben, so dass der betreffende Agonist weniger leicht gerochen wird. Dies kann insbesondere in den Fällen sinnvoll sein, in denen ein Agonist einen störenden Geruch entwickelt, jedoch nur mit unverhältnismäßig hohem Aufwand aus der Luft oder einem anderen Trägermedium entfernt werden kann.

Zum Herstellen eines erfindungsgemäßen Rezeptors wird ein Verfahren angegeben, das die Schritte umfasst:
a) Einbringen eines erfindungsgemäßen Vektors in eine Zelle, und
b) Einstellen von Bedingungen, so dass der Nucleinsäureabschnitt des Vektors, der für den Rezeptor codiert, in der Zelle exprimiert wird.

Mit Hilfe dieses Verfahrens ist es möglich, ein erfindungsgemäßes Expressionssystem und damit den erfindungsgemäßen Rezeptor und gegebenenfalls auch einen erfindungsgemäßen Biosensor herzustellen. Der erfindungsgemäße Rezeptor kann aus den Zellen des Expressionssystems weiter aufgereinigt werden.

Im Zusammenhang mit der vorliegenden Erfindung wird ein Verfahren beschrieben zum Nachweisen
1) eines Agonisten der Formel I, vorzugsweise eines Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd, und/oder
2) eines Antagonisten gegen das Binden eines Agonisten gemäß 1) an den Rezeptor, vorzugsweise n-Undecanal,
in einer Probe, wobei das Verfahren die Schritte umfasst:
a) Bereitstellen eines erfindungsgemäßen Rezeptors,
b) Inkontaktbringen des Rezeptors mit der zu untersuchenden Probe und Einstellen von Bedingungen, bei denen ein gegebenenfalls in der Probe enthaltener Agonist und/oder Antagonist spezifisch an den Rezeptor bindet, und
c) Überprüfen, ob ein Agonist und/oder ein Antagonist eine spezifische Bindung mit dem Rezeptor eingegangen ist.

Der erfindungsgemäße Rezeptor kann insbesondere in Form eines Expressionssystems bereitgestellt werden, in dem der Rezeptor in Wirkverbindung mit einer Signaltransduktionskaskade steht. In diesem Fall kann eine spezifische Bindung eines Agonisten an den Rezeptor durch das Einschalten der betreffenden Signaltransduktionskaskade überprüft werden, während das spezifische Binden des Antagonisten durch das Ausbleiben eines solchen Einschaltens der Signaltransduktionskaskade bei Anwesenheit eines Agonisten überprüft werden kann. Ein solches Expressionssystem kann jedoch auch künstlich nachgebildet werden, indem der Rezeptor und die übrigen Bestandteile der Signaltransduktionskaskade *in vitro* zusammengestellt werden.

Der Rezeptor kann jedoch auch als aufgereinigtes Protein vorliegen, das an die Oberfläche eines festen Trägers, insbesondere einer Metalloberfläche, gebunden ist. In diesem Fall kann-sich durch das spezifische Binden eines Agonisten und/oder eines Antagonisten an den Rezeptor eine physikalische Oberflächeneigenschaft ändern. Dies kann beispielsweise die Änderung der Oberflächen-Plasmon-Resonanz sein, wie sie in einem Biacore-Verfahren bestimmt werden kann.

Ebenfalls möglich ist es, den Rezeptor und/oder einen Agonisten und/oder einen Antagonisten mit einem Fluoreszenzfarbstoff zu versehen und die durch die spezifische Bindung des Agonisten und/oder des Antagonisten an den Rezeptor hervorgerufene Änderung der Fluoreszenz zu bestimmen.

Insbesondere kann der Rezeptor als Fusionsprotein mit einem *green fluorescent protein*-(GFP-)-Anteil vorliegen, so dass sich die Fluoreszenz des Fusionsproteins bzw. des GFP-Anteils des Fusionsproteins bei spezifischer Bindung eines Agonisten und/oder eines Antagonisten entsprechend ändert. Dem Fachmann sind solche Untersuchungsverfahren unter der Bezeichnung "FRET-Assays" (*Fluorescence resonance energy transfer* assays) bekannt. Auf vergleichbare Weisekann der Rezeptor auch als Fusionsprotein mit einem Luciferase-Anteil vorliegen, so dass sich die Lumineszenz des Fusionsproteins bzw. des Luciferase-Anteils des Fusionsproteins bei spezifischer Bindung eines Agonisten und/oder eines Antagonisten ändert ("BRET-Assay").

Ein erfindungsgemäßes Verfahren zum Nachweisen des Antagonisten in einer Probe umfasst folgende Schritte:
a) Bereitstellen eines Rezeptors, der einen Proteinabschnitt mit einer Aminosäuresequenz gemäß SEQ ID NO.4 oder SEQ ID NO. 8, oder mit einem Proteinabschnitt mit einer Aminosäure-Sequenzhomologie von mehr als 70% zu einem Proteinabschnitt mit einer Aminosäuresequenz gemäß SEQ ID NO.4 oder SEQ ID NO. 8 besitzt,
b) Inkontaktbringen des Rezeptors mit der zu untersuchenden Probe und Einstellen von Bedingungen, bei denen ein gegebenenfalls in der Probe enthaltener Antagonist spezifisch an den Rezeptor bindet,
c) Inkontaktbringen des Rezeptors mit einem Agonisten der Formel I, vorzugsweise einem Agonisten ausgewählt aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-butylphenoxy)acetaldehyd ausgewählten Agonisten, um dem Agonisten ein spezifisches Binden an den Rezeptor zu ermöglichen, falls kein Antagonist spezifisch an den Rezeptor gebunden ist, und
d) Überprüfen, ob der Agonist und/oder ein Antagonist eine spezifische Bindung mit dem Rezeptor eingegangen ist.

Der Fachmann erkennt, dass die Schritte b) und c) gleichzeitig oder in einer beliebigen Reihenfolge nacheinander durchgeführt werden können, vorausgesetzt, dass Schritt c) vor oder gleichzeitig mit Schritt d) durchgeführt wird. Insbesondere können folgende Verfahrensausführungen zweckmäßig sein:
In einer bevorzugten Ausführungsform wird zunächst der erfindungsgemäße Rezeptor bereitgestellt. Hierzu kann sich der Fachmann der Möglichkeiten bedienen, die oben für ein erfindungsgemäßes Verfahren zum Nachweisen eines Agonisten und/oder Antagonisten beschrieben wurden. Anschließend wird der Rezeptor mit der gegebenenfalls antagonistenhaltigen Probe in Kontakt gebracht. Dabei werden solche Bedingungen eingestellt, unter denen ein Antagonist spezifisch an den Rezeptor binden könnte, so er denn in der Probe vorhanden ist. Nach dem Inkontaktbringen mit dem Antagonisten wird der Rezeptor mit einem Agonisten in Kontakt gebracht. Hierbei werden die Bedingungen so eingestellt, dass der Agonist an den Rezeptor spezifisch binden kann, wenn kein Antagonist an den Rezeptor spezifisch gebunden hat. Abschließend wird überprüft, ob der Agonist an den Rezeptor spezifisch gebunden hat. Dies kann beispielsweise dadurch geschehen, dass das Einschalten einer Signaltransduktionskaskade überprüft wird, mit der der Rezeptor in Wirkverbindung steht. Wenn der Agonist an den Rezeptor gebunden hat, dann liegt der Antagonist wahrscheinlich nicht in einer Konzentration in der zu untersuchenden Probe vor, bei der er das spezifische Binden des Agonisten verhindern könnte. Insbesondere kann es möglich sein, das Maß der Agonistenbindung an den Rezeptor zu bestimmen und hieraus - gegebenenfalls nach einer entsprechenden Kalibrierung - auf die Konzentration des Antagonisten in der zu untersuchenden Probe zu schließen.

In einer bevorzugten Abwandlung des Verfahrens wird der Rezeptor zuerst mit dem Agonisten und anschließend mit der den Antagonisten gegebenenfalls enthaltenden Probe in Kontakt gebracht. In diesem Fall kann aus der Abnahme des Maßes, in dem der Agonist spezifisch an den Rezeptor gebunden ist, auf die Anwesenheit des Antagonisten und gegebenenfalls auch auf dessen Konzentration in der zu untersuchenden Probe geschlossen werden.

Die Erfindung wird nachfolgend anhand der Figuren und der Beispiele näher erläutert.
Fig. 1 stellt das Ergebnis einer Gelelektrophorese nach der RT-PCR dar. Die mRNA von hOR 17-4 wurde nachgewiesen (A). Als Positivkontrolle diente humane genomische DNA (B) und als Negativkontrolle ein Teil der vor der cDNA-Synthese angereicherten mRNA. GAPDH dient der Überprüfung der cDNA-Synthese.
Fig. 2 zeigt die Struktur des chimären Vektoplasmides hOR17-4 (Ausschnitt).
Fig. 3 stellt im zufällig gewählten Sichtfeld die vorübergehende Induktion von Ca²⁺-Signalen in HEK293-Zellen, die transient mit pOR17-4 transfiziert worden waren, durch die komplexe Geruchstoffmischung Henkel 100 dar.
Fig. 4 stellt das Fluoreszenzverhältnis (f₃₄₀/f₃₈₀) der in Figur 3 durch einen Kreis markierten Zellen als Funktion der Zeit dar.
Fig. 5 stellt die Unterteilung der Henkel 100 Geruchsstoffmischung dar, die zur Identifizierung des hOR17-4 Agonisten Cyclamal führten (links). Rechts ein typisches Antwortmuster auf die Applikation eines hOR17-4 nicht aktivierenden Stoffes (Citrusal) und von Cyclamal.
Fig. 6 stellt eine Liste aktivierender und nicht-aktivierender Substanzen dar.
Fig. 7 stellt die Effizienz-Klassifikation hOR17-4 aktivierender Komponenten dar, wie sie durch die Normalisierung deren gemittelter Antwortamplitude auf diejenige der ATP-Antwort erhalten wurde (n > 15 ± SEM).
Fig. 8 stellt den jeweiligen Protzensatz (mit Ca²⁺-Signalen) antwortender Zellen auf die Applikation von verschiedenen Geruchsstoffen [jeweils bei 500 µM oder 50 µM] hin dar. Normiert wurde auf die als 100 % definierte Zahl der Zellen, die durch Cyclamal in hoher Konzentration [~ 5 mM] zu einer Zellantwort stimuliert wurden (Cyclamal reponsive Zellen in jeder Experimentreihe : n > 35).
Fig. 9-15 zeigen fluororatiometrische Aufzeichnungen von pOR17-4 transfizierten HEK293-Zellen. Der intrazelluläre Ca²⁺-Spiegel verschiedener Fura-2 geladener Zellen wird als Fluoreszenzverhältnis (f₃₄₀/f₃₈₀) als Funktion der Zeit dargestellt. Wenn nicht durch den Applikations-Balken anders angezeigt, wurden alle getesteten Komponenten [500 µM] und ATP [200 µM] für 5 Sekunden appliziert.

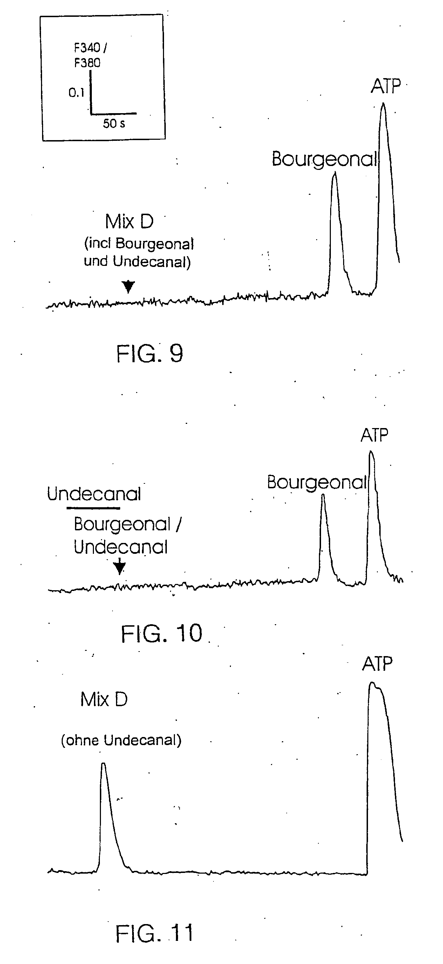

Fig. 9 stellt dar, dass die 10 Komponenten enthaltende Henkel Mix D-Mischung hOR17-4 exprimierende Zellen nicht aktiveren kann, die durch Bourgeonal alleine aktiviert werden können.
Fig. 10 zeigt, dass die Vorinkubation mit Undecanal für 50 Sekunden die hOR17-4 Rezeptoraktivierung durch Bourgeonal inhibiert.
Fig. 11 zeigt, dass die Bestandteile einer Henkel Mix D-Mischung aus der n-Undecanal entfernt wurde hOR17-4 exprimierende HEK293 Zellen aktivieren kann.

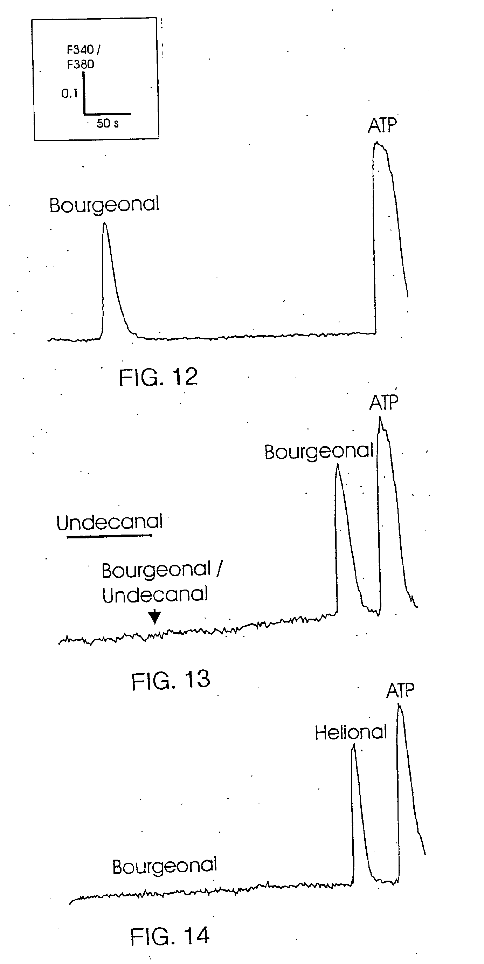

Fig. 12 zeigt, dass Bourgeonal auch den hOR17-4 Rezeptor der vollen Länge aktiviert.
Fig. 13 zeigt, dass die Vorinkubation mit n-Undecanal die Rezeptoraktivierung des hOR17-4 in voller Länge inhibiert.
Fig. 14 zeigt, dass ein weiterer humaner olfaktorischer Aldehydrezeptor (hOR17-40) nicht durch Bourgeonal aktiviert werden kann.

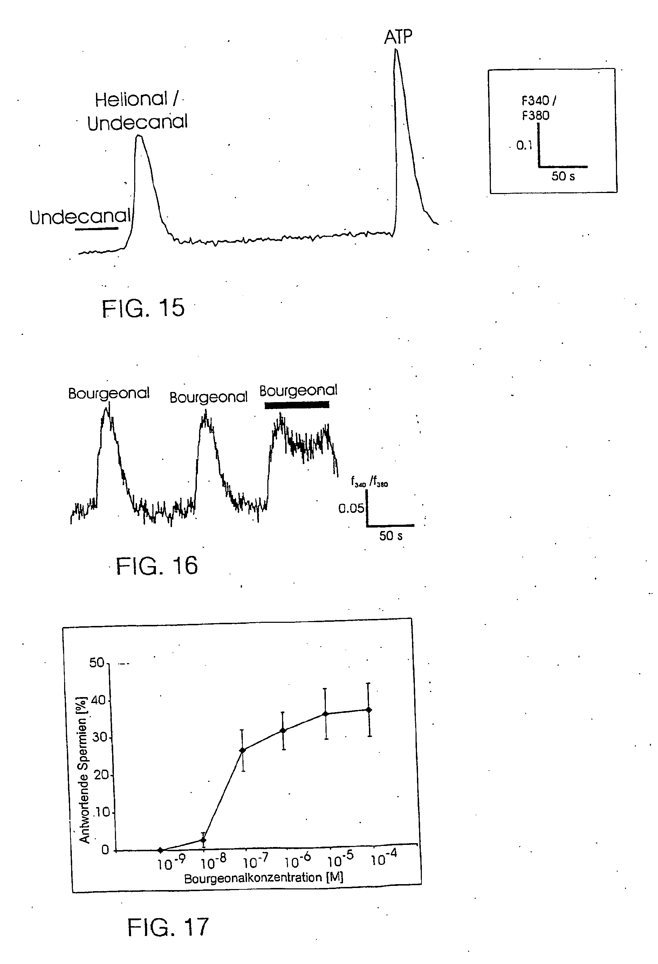

Fig. 15 stellt dar, dass die Helionalantwort von hOR17-40 nicht durch die Vorinkubation mit n-Undecanal inhibiert werden kann.
Fig. 16 stellt eine repräsentative flurororatiometrische Aufzeichnung des Kopfes und Mittelstückes eines einzelnen humanen Spermiums dar. Bourgeonal induziert reproduzierbare Ca²⁺-Antworten in 36 % aller Spermien. Im Gegensatz zum heterolog exprimierten hOR17-4-Rezeptor führt längere Bourgeonal-Stimulation zu entspechend längeren Ca²⁺-Signalen.
Fig. 17 stellt den Prozentsatz von Spermien mit Ca²⁺ -Signalen in Abhängigkeit der Konzentration des applizierten Bourgeonals dar.

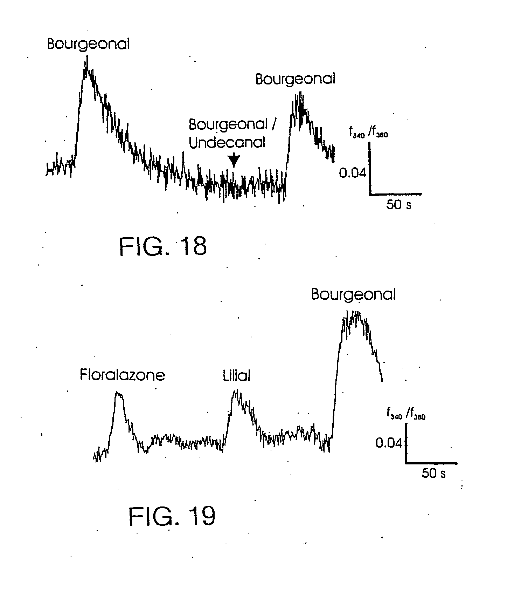

Fig. 18 stellt die Supprimierung der Kalziumantwort durch Co-Applikation von Bourgeonal und n-Undecanal dar.
Fig. 19 stellt die Kalziumantwort eines einzelnen Spermiums auf Floralazon, Lilial und Bourgeonal dar.

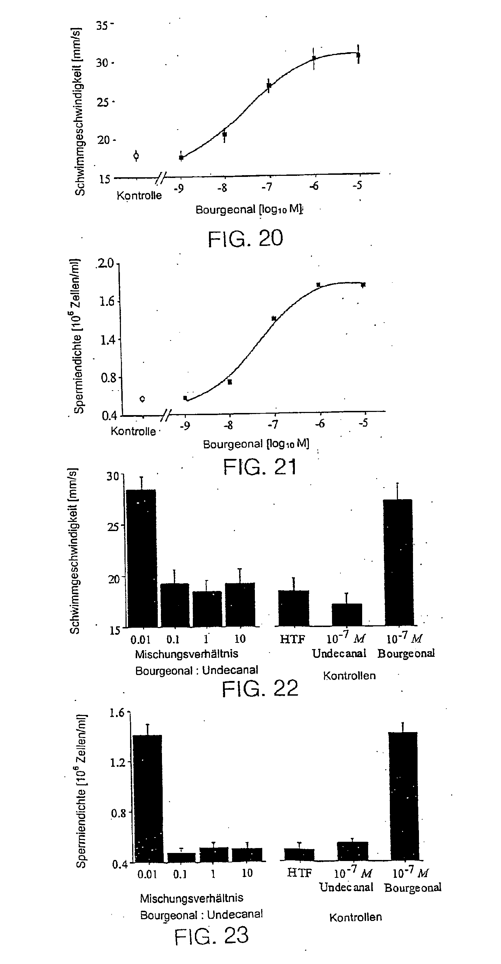

Fig. 20 stellt die durchschnittliche Schwimmgeschwindigkeit von Spermien in Abhängigkeit von der Bourgeonal-Konzentration (logarithmische Skala) dar.
Fig. 21 stellt die Spermiendichte in Abhängigkeit von der Bourgeonal-Konzentration (logarithmische Skala) dar.
Fig. 22 stellt den Effekt von n-Undecanal auf die von Bourgeonal abhängige Schwimmgeschwindigkeit humaner Spermien dar.
Fig. 23 stellt den Effekt von n-Undecanal auf die von Bourgeonal beeinflusste Spermiendichte humaner Spermien vor einer Applikationskanüle dar.

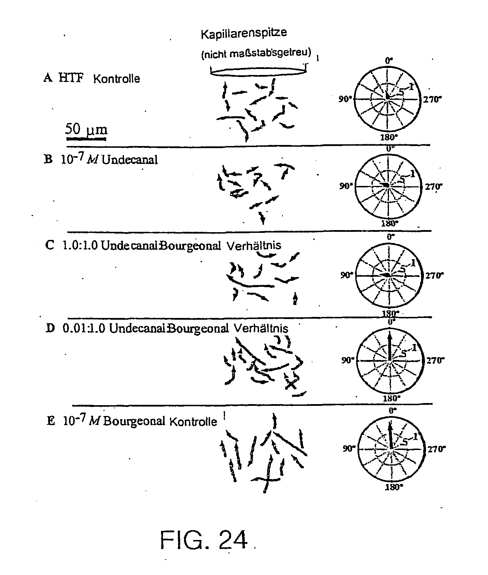

Fig. 24 stellt den Einfluss von Bourgeonal und n-Undecanal sowie Mischungen der beiden Substanzen auf die Schwimmrichtung humaner Spermien dar.

### Beispiel 1: Definitionen

Die verwendeten Sequenzen sind in beigefügtem Sequenzprotokoll im einzelnen wiedergegeben.

### Beispiel 2: Expression der hOR17-4-messenaer RNA in humanen Hoden

Die RNA aus dem Hoden eines 69 Jahre alten Mannes wurde mit Trizol-Reagenz (Invitrogen, Carlsbad, CA) isoliert und DNAseI-verdaut, bevor die polyA+ mRNA durch Bindung an Oligo-dT-beschichtete paramagnetische Partikel (Dynal, Oslo, Norwegen) isoliert wurde. cDNA wurde unter Verwendung von MMLV-Reverser Transcriptase (Invitrogen, Carlsbad, CA) und Oligo (dT₁₂₋₁₈)-Primern hergestellt. Die cDNA wurde anschließend in jeweils einem PCR-Ansatz amplifiziert. Die Amplifikation wurde mit etwa 1ng cDNA und spezifischen Primern für hOR17-4 (SEQ ID NO. 1 und SEQ ID NO. 2) durchgeführt. Als Positivkontrolle dienten 100 ng genomischer DNA und als Negativkontrolle die gleiche Konzentration mRNA, wie sie in die RT-PCR eingesetzt wurde. Die Amplifikation wurde mit 35 Zyklen (94 °C für 1 Minute, 60 °C für 1 Minute, 72 °C für 1 Minute) ausgeführt und 2,5 Units *Taq-*Polymerase + 30 pMol von jedem Primer wurden gemäß den Herstellerempfehlungen (Invitrogen, Carlsbad, CA) hinzugefügt.

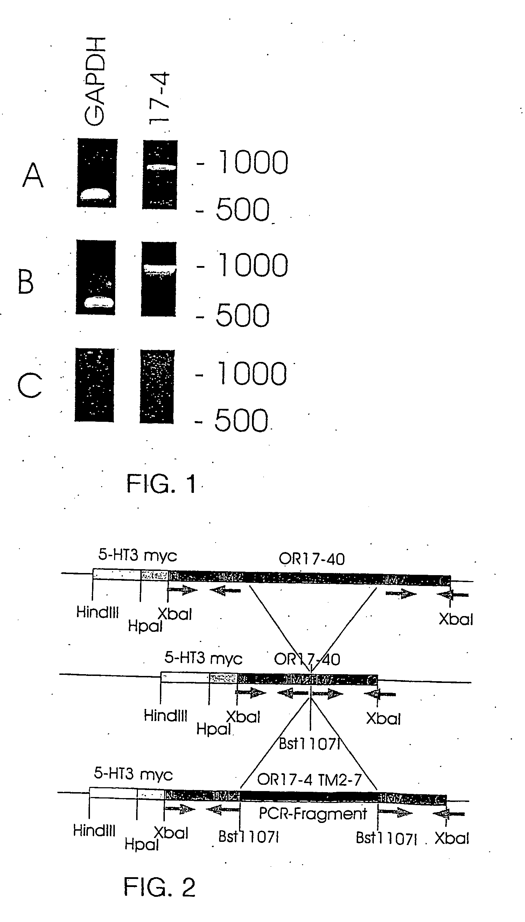

Fig. 1A zeigt die auf 1,5 %iges Agarose-Gel aufgetragen Produkte der einzelnen PCR-Ansätze. Die Spaltenbeschriftung gibt wieder, für welches hOR-Gen die jeweils verwendeten Primerpaare spezifisch waren. Die für hOR17-4 spezifische PCR hat ein Amplifikationsprodukt von etwa 982 bp ergeben. Diese Größe war gemäß SEQ ID NO. 3 zu erwarten.

Fig. 1 B zeigt das Ergebnis der Positivkontrolle, Fig. 1C das der Negativkontrolle.

Die Bildung des für hOR17-4 spezifischen Amplifikationsprodukts zeigt, dass dieses Gen in humanem Hodengewebe exprimiert wird. Auf entsprechende Weise wurde hOR17-4-mRNA auch in Nebenhodengewebe nachgewiesen.

### Beispiel 3: Darstellung des Konstruktes pOR17-4

Es wird vermutet, dass die Transmembrandomänen III-VI eine Bindungstasche zum Binden von Agonisten bilden (s. S. Firestein, Nature 413, 211-218 (2001)). Um diese Vermutung für den Rezeptor hOR17-4 zu überprüfen wurde ein für die vermuteten Transmembrandomänen III bis VI codierender Abschnitt der für hOR17-4 codierenden cDNA in einem Shuttle-Expressions-Vektor (pOLF-Express) kloniert. Der Expressionsvektor pOLF-Express umfasst die für die N- und C-terminalen Regionen von hOR17-40 codierenden DNA-Abschnitte (C. H. Wetzel et al., Journal of Neuroscience 19, 7426-7433 (1999)). Das resultierende chimäre Rezeptorprotein sollte die Agonistenspezifität des vollständigen Rezeptors hOR17-4 besitzen.

Nachfolgend (vgl. Beispiel 4) wird auch die Konstruktion eines Expressionsvektors für das nicht-chimäre Rezeptorprotein hOR17-4 beschrieben. Es hat sich tatsächlich herausgestellt, dass sich das Bindungsverhalten gegenüber den getesteten Agonisten und Antagonisten zwischen dem chimären Rezeptorprotein und dem nicht-chimären Rezeptorprotein nicht signifikant unterscheidet. Deshalb wird im Folgenden nicht mehr zwischen dem nicht-chimären Rezeptorprotein und dem chimären Rezeptorprotein unterschieden.

Zur Herstellung des Expressionsvektors pOLF-Express wurde das 5'- und das 3'-Ende der hOR17-40-cDNA unter Verwendung spezifischer Primer (für das 5'-Fragment: SEQ ID NO. 9 und SEQ ID NO. 10; für das 3'-Fragment: SEQ ID NO. 11 und SEQ ID NO. 12) mit dem Plasmid pOR17-40 (Wetzel *et al.,* a.a.O.) amplifiziert. Zur Generierung chimärer DNA wurden die PCR-Produkte ligiert und das gewünschte heterodimere Fragment mittels PCR amplifiziert. Das so erhaltene Fragment wurde nachfolgend in den Vektor pRC/CMV cloniert, nachdem dieser mit HindIII und Apal verdaut und die überhängenden Enden mit Klenow-Enzym aufgefüllt worden waren. In dem resultierenden Vektor pOLF-Express liegt zwischen dem 3'- und dem 5'-seitigen Endabschnitt der hOR17-40 cDNA eine Bst 11071-Schnittstelle.

Die für die vermuteten Transmembrandomänen III - VI codierende Region wurde durch PCR mit den Primern gemäß SEQ ID NO. 5 und SEQ ID NO. 6 mit humaner genomischer DNA als Vorlage (Template) amplifiziert. Das resultierende 626 bp lange PCR-Fragment gemäß SEQ ID NO. 7 wurde in die Bst 1107I-Schnittstelle von pOLF-Express subcloniert.

Figur 2 zeigt schematisch die Struktur der wesentlichen bei der Herstellung von pOLF-Express und pOR17-4 anfallenden Konstrukte (von oben nach unten):
1. pOR17-40 (Ausschnitt) mit der vollständigen cDNA von hOR17-40
2. pOLF-Express mit den zusammengefügten C- und N-terminalen Bereichen des pOR17-40 (Ausschnitt)
3. pOR 17-4 (Ausschnitt)

### Beispiel 4: Erstellung des Vektors ohne künstliche Sequenzen(pOR17-4v)

Um einen Expressionsvektor für das gesamte hOR17-4-Gen ohne geänderte Aminosäuresequenz zu erhalten, wurde das 982 bp lange PCR-Produkt (SEQ ID NO. 3, Herstellung vergleiche Beispiel 2) über stumpfe Enden in den Hindlll/Xbal verdauten Vektor pRC/CMV (Invitrogen, Carlsbad, CA), dessen überhängende Enden mit Klenow-Enzym aufgefüllt worden waren, einkloniert.

Alle Modifikationen in Beispiel 3 und 4 wurden unter Standardbedingungen mit einer Mischung von *Taq-* und *Pfu*-Polymerase (19:1) gemäß den Herstellerempfehlungen durchgeführt.

### Beispiel 5: Transiente Transfektion von pOR17-4 oder pOR17-4v in HEK293-Zellen

HEK293-Zellen wurden in MEM (Life Technologies, Gaithersburg, MD), das mit 10 % hitze-inaktiviertem fetalem Kälber-Serum angereichert war, in einem Inkubator bei 37 °C in einer Atmosphäre aus 95 % befeuchteter Luft und 5 % CO₂ kultiviert. Semikonfluente Zellen wurden in 35 mm Gefäßen (Falcon) mittels Calciumphosphat-Präzipitation (Zufall et al., PNAS 90 (1993), 9335-9339) unter Verwendung jeweils des Plasmids pOR17-4 bzw. pOR17-4v transfiziert. Die Transfektionseffizienz - typischerweise 10 % - wurde histochemisch mit dem Reporterplasmid pCH110 (Pharmacia, Uppsala, Schweden) überprüft, das für β-Galactosidase codiert. Messungen wurden 48 bis 72 Stunden nach der Transfektion durchgeführt. Die Transfektionseffizienz kann mit einem für GFP, einem grün fluoreszierenden Protein, codierenden Reporterplasmid ebenfalls überprüft werden.

### Beispiel 6: Messung der Rezeptoraktivierung in HEK293-Zellen

Die Rezeptor-Aktivierung in HEK293-Zellen führt zu einem vorübergehenden Anstieg der cytosolischen Ca²⁺-Konzentration, die auf der Einzelzell-Ebene unter Verwendung des Ca²⁺-sensitiven Farbstoffes Fura-2 (Fura-2 AM, Molecular Probes Europe BV, Leiden, Niederlande; Ladekonzentration 3-4 µg/ml; Ladezeit ca. 30 min) und ratiofluorometrischen Bildgebungstechniken (Imaging-Experimente) detektiert wurden.

Die Ca²⁺-imaging-Experimente wurden auf dem Träger eines invertierten Zeiss-Mikroskopes durchgeführt, das für die ratiofluorometrische Bildgebung mit einer Xenon-Bogenlampe, einem Vielfach-Wellenlängen-Illuminations-System POLYCH-ROM II zur Anregung, einer gekühlten charge-coupled device (CCD)-Kamera und einer WinNT-basierten T.I.L.L.-Vision-Software zum Sammeln und raum- und zeitabhängigen Quantifizieren Ca²⁺-abhängiger Fluoreszenz-Signale (F340/F380-Verhältnis) ausgestattet war. HEK293-Zellen wurden mit 32-facher, Spermazellen mit 63-facher Vergrößerung beobachtet.

### Beispiel 7: Applikation von Geruchsstoffen auf HEK293- und Spermazellen

HEK293- und Spermazellen wurden unter Verwendung eines speziellen Applikations-Systems, das es gestattete, vorübergehend alle Zellen im Blickfeld aus einem von sechs benutzergewählten Kapillarröhrchen zu überspülen, Geruchs- und/oder Wirkstoffen (Testlösungen) ausgesetzt. Der Wechsel zwischen den Testlösungen erfolgte nahezu sofort; auch trat im Wesentlichen keine Wirkungsverzögerung nach dem Wechsel auf, weil die Röhrchenspitzen zum optischen Feld eng benachbart waren. Zwischen den Applikationen von Testlösungen überspülte ein Pufferlösungsstrom kontinuierlich alle Zellen im Gefäß, um jedwede potentielle Hintergrund-Anreicherung von Testlösungen (Geruchs- bzw. Wirkstoffen) zu minimieren. Alle Lösungen wurden bei Raumtemperatur appliziert. Alle verwendeten Geruchsstoffe stammten von Dr. T. Gerke, Henkel KGaA, Düsseldorf, FRG, andere Wirkstoffe wurden von Sigma gekauft. Zur Zusammensetzung der Henkel 100-Mischung vergleiche C. H. Wetzel et al., Journal of Neuroscience 19, 7426-7433 (1999).

### Beispiel 8: Überprüfung des Expressionssystems

Die Zuverlässigkeit des Expressionssystemes wurde überprüft, indem Zellen mit dem bei C. H. Wetzel et al., Journal of Neuroscience 19, 7426-7433 (1999) beschriebenen pOR17-40 transfiziert wurden. Auf das Applizieren von Helional erhöhte sich vorübergehend der cytosolische Ca²⁺-Spiegel in 2 - 5 % aller getesteten Zellen (n=168). Der Unterschied zu der vorhergesagten Transfektionsrate (ungefähr 10%, vergleiche oben Beispiel 5) kann vermutlich durch die Abwesenheit von olfaktorisch-spezifischen Co-Faktoren, die für die angemessene Membranlokalisierung benötigt werden, und/oder ineffiziente Kopplung an die Transduktionsmaschinerie in nicht-olfaktorischen Zellen erklärt werden.

### Beispiel 9: Bestimmung der Agonistenspezifität

Alle auf die potentielle Aktivierung von hOR17-4 untersuchten Geruchsstoffe wurden wenigstens in drei verschiedenen Reihen von Transfektionsexperimenten getestet. Als Liganden wurden alle Komponenten angesehen, die zu klaren Ca²⁺-Antworten in mehreren verschiedenen Experimenten (n>7) führten, während sie keine Ca²⁺-Signale in Gefäßen mit untransfizierten Zellen auslösten. Geruchsstoffe, die nicht in wenigstens 2 % aller HEK293-Zellen messbare Ca²⁺-Konzentrationssteigerung auslösten, wurden nicht zu den hOR17-4-spezifischen Agonisten gezählt, ebenso diejenigen wenigen der untersuchten Komponenten, die ein unspezifisches Ca²⁺-Signal in fast jeder Zelle hervorriefen.

### Beispiel 10: Suche eines spezifisch an hOR17-4 bindenden Agonisten

Zur Suche eines spezifischen Agonisten für hOR17-4 wurde eine komplexe Mischung von 100 verschiedenen Geruchsstoffen (Henkel 100) verwendet, s.o. Beispiel 7, die aromatische und kurzkettige aliphatische Komponenten mit verschiedenen funktionellen Gruppen beinhaltete. Das Ligandenscreening wurde gemäß den Beispielen 6, 7 und 9 durchgeführt.

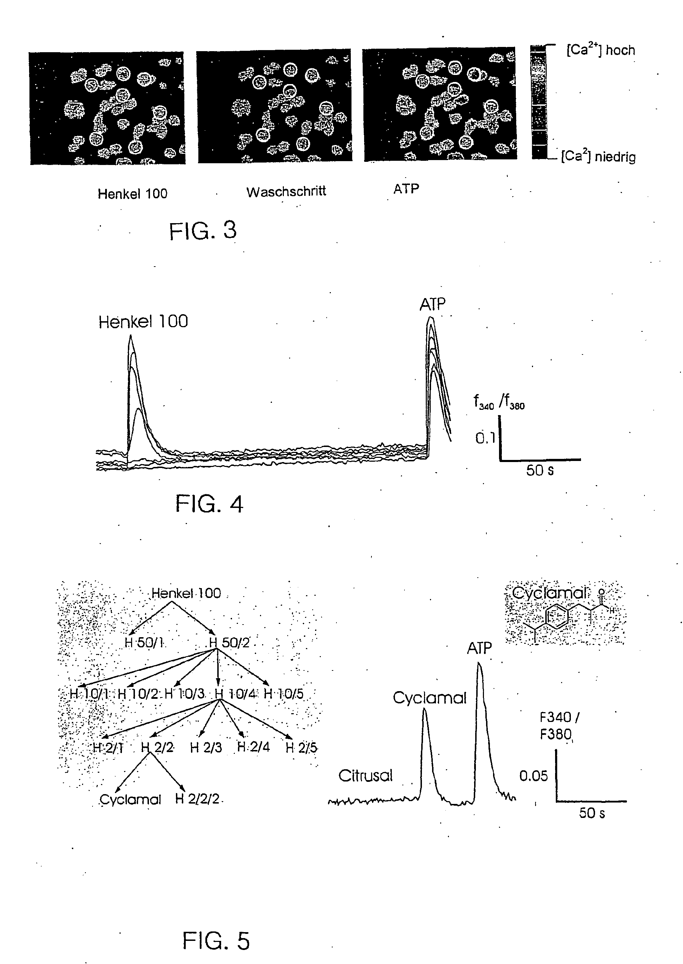

Die 1:1000 in normaler Ringer-Lösung verdünnte Henkel 100-Mischung und ihre Teilmischungen induzierte vorübergehende cytosolische Ca²⁺-Konzentrations-steigerungen (Antworten) in 2 - 5 % aller getesteten Zellen (vergleiche Fig. 3. Diese stellt im zufällig gewählten Sichtfeld die Induktion von vorübergehenden Ca²⁺-Signalen in Zellen, die transient mit pOR17-4 transfiziert worden waren, durch die komplexe Geruchstoffmischung Henkel 100 dar. Bis zu 5 % aller HEK293-Zellen reagierten mit einem Anstieg der cytosolischen Ca²⁺-Konzentration. Die Ca²⁺-Konzentrationsveränderungen werden per Pseudo-Farben dargestellt. Sowohl Henkel 100 [1:1000] und ATP [200 M] wurden für 5 Sekunden appliziert). Es wurden keine Ca²⁺-Signale in nicht-transfizierten HEK293-Zellen beobachtet, auch nicht nach Applizieren einer 10-fach höheren Geruchsstoffkonzentration. Die Ca²⁺-vermittelte Antwort auf den Geruchsstoff wird charakterisiert durch eine kurze Verzögerung, einen Anstieg von wenigen Sekunden und ein ähnliches Abklingen (vergleiche Fig. 4, Henkel 100 (1:1000) und ATP (200 µM) wurden jeweils 5 Sekunden lang appliziert). Auch lang anhaltende Applikationen der Geruchsstoffmischung führten zu vorübergehenden Ca²⁺-Antworten in 7 von 7 Zellen. Der Gesamtprozentsatz antwortender Zellen wurde als repräsentativer Mittelwert aus einer großen Zahl von verschiedenen Experimenten und Transfektionen bestimmt. Um Informationen über den physiologischen Zustand der HEK293-Zellen und ihre Fähigkeit, Phospholipase-C-vermittelte Ca²⁺-Signale zu produzieren, zu erlangen, wurde 200 µM ATP am Ende jedes Experimentes appliziert.

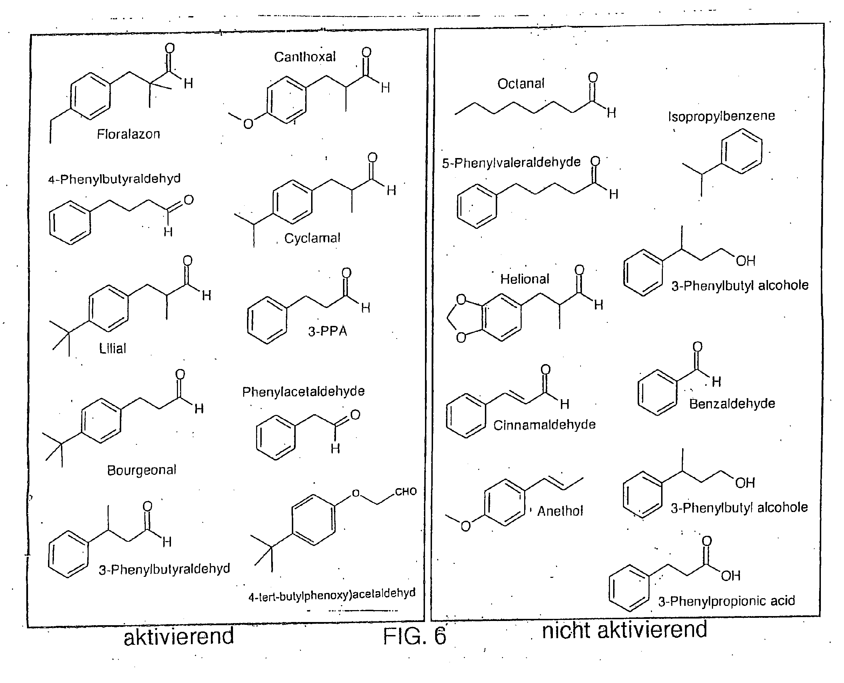

Um den oder die tatsächlichen Agonisten für hOR17-4 innerhalb der Henkel 100-Mischung zu bestimmen, wurde die komplexe Geruchsstoffmischug in Portionen von 50, 10 und 2 Komponenten unterteilt, die Portionen wurden jeweils auf ihre Wirkung auf pOR17-4-transfizierte Zellen hin untersucht (vergleiche Fig. 5). Auf diesem Weg wurde ein Agonist identifiziert: Cyclamal (vergleiche Liste der aktiven Komponenten, Fig. 6).

In den meisten responsiven Zellen führt eine einmalige Applikation von Cyclamal zu einer vollständigen und langanhaltenden Desensitisierung der Geruchsstoff-Antwort. Demgegenüber blieb die ATP-Antwort unverändert bestehen. Der schnelle Desensitisierungsprozess, der häufig bei HEK/OR-Expressionssystemen (vergleiche D. Krautwurst, K. W. Yau, R. R. Reed, Cell 95, 917-926 (1998), Zhang et al., Proceedings of the National Academy of Sciences of the United States of America 94 (1997), 12162-12167) beobachtet wird, dürfte auf der Rezeptor- oder G-Protein-Ebene seine Ursache haben.

### Beispiel 11: Bestimmung weiterer spezifischer Agonisten und eines Agonistenstrukturmodelles

Das "combinatorial code model" der Geruchsstoff-Detektion (B. Malnic, J. Hirono, T. Sato, L. B. Buck, Cell 96, 713-723 (1999).) schlägt vor, dass ein einzelner Geruchsststoffrezeptor vielfältige Geruchsstoffe mit unterschiedlichen Affinitäten erkennt. Da in Beispiel 10 nur eine verhältnismäßig geringere Anzahl von Geruchsstoffen untersucht wurden, sollten weitere Agonisten für hOR17-4 gesucht werden. Dementsprechend wurde bestimmt, welche strukturellen Komponenten ein Molekül besitzen muss, um als Agonist für hOR17-4 zu dienen. Dazu wurde von der räumlichen Struktur von Cyclamal ausgegangen, um andere mögliche Agonisten über Strukturanalogie abzuleiten. Die Kandidatenmoleküle wurden in einer Konzentration von etwa 500 µM gemäß den Beispielen 6, 7, 9 und 10 eingesetzt.

In einem ersten Schritt wurde zu definieren versucht, welches die minimalen Anforderungen zur Aktivierung von hOR17-4 sind. Das aromatische Isopropylbenzol ohne die Aldehydgruppe und ohne die verbindende Kohlenstoffkette induzierte keine Rezeptoraktivität, aber sowohl Phenylbutylaldehyd (3-PBA) und 3-Phenylpropanal (3-PPA), beide ohne Substituent in Paraposition und voneinander nur durch eine einzelne Methylgruppe unterschieden, führten zu klaren Ca²⁺-Antworten. Als nächstes wurde die Aldehydgruppe von 3-PPA durch eine Carboxygruppe und die von 3-PBA durch eine Hydroxygruppe ausgetauscht. Sowohl 3-Phenylpropionsäure als auch 3-Phenylbutylalkohol aktivierten den Rezeptor nicht, ebenso wie Anethol. Zusammengefasst legen diese Daten nahe, dass eine Aldehydgruppe zur Aktivierung von hOR17-4 notwendig ist.

Um die Bedeutung des aromatischen Ringes zu bestimmen und um die Möglichkeit auszuschließen, dass der Rezeptor ein wenig spezifischer Aldehydrezeptor ist, wurde Octanal getestet, ein aliphatischer Geruchsstoff, von dem bekannt ist, dass er den Ratten-I7-Rezeptor (D. Krautwurst, K. W. Yau, R. R. Reed, Cell 95, 917-926 (1998); H. Zhao et al., Science 279, 237-242 (1998)) stimuliert. Es stellte sich heraus, dass Octanal den Rezeptor hOR17-4 nicht aktiviert.

Mehrere Studien zu anderen Rezeptoren weisen darauf hin, dass die Länge der Kohlenstoffkette eine wichtige strukturelle Eigenschaft für die Geruchsstoff-Rezeptorinteraktion ist (D. Krautwurst, K. W. Yau, R. R. Reed, Cell 95, 917-926 (1998); B. Malnic, J. Hirono, T. Sato, L. B. Buck, Cell 96, 713-723 (1999)). Dementsprechend wurden Komponenten getestet, die vom aktiven 3-PPA abgeleitet wurden und in der Entfernung zwischen dem aromatischen Ring und der Aldehydgruppe variierten. Die Applikation von Phenylacetaldehyd oder 4-Phenylbutylaldehyd (4-PBA) induzierte deutliche Ca²⁺-Signale, aber der kurzkettige Benzaldehyd und der langkettige 5-Phenylvaleraldehyd bewirkten keine messbare Antwort.

Zusammengenommen legen diese Daten nahe, dass eine Aldehydgruppe, die mit einer aromatischen Struktur durch eine Kette von zwischen 1 bis 3 Atomen verbunden ist, eine Voraussetzung für die Agonistenerkennung durch hOR17-4 ist.

Floralazon, Lilial und Bourgeonal unterscheiden sich von Cyclamal durch die Anwesenheit bzw. Abwesenheit von Methylsubstituenten an verschiedenen Atomen der verbindenden Kette. Diese Substituenten beeinträchtigen nicht die Fähigkeit der Substanzen, den Rezeptor zu aktivieren, was darauf hinweist, dass hOR17-4 verschiedene Grade der Methylierung toleriert. Sogar (4-tert-Butylphenoxy)acetaldehyd (4-PBAA), das statt eines Kohlenstoffatoms in der verbindenden Kette ein Sauerstoffatom enthält, erzeugte ein Ca²⁺-Signal.

Es wird berichtet, dass das Einführen von Doppelbindungen in eine gegebene Komponente die Anpassung des Rezeptors an ein versteiftes Molekül einer gegebenen Länge inhibiert (R. C. Araneda, A. D. Kini, S. Firestein, Nature Neuroscience 3, 1248-1255 (2000). Cinnamaldehyd, der sich von 3-PPA durch die Einführung einer einzelnen Doppelbindung unterscheidet, ist vollständig inaktiv. Helional, ein Geruchsstoff, von dem bekannt ist, dass er den einzigen bisher funktionell exprimierten humanen Geruchsrezeptor (hOR17-40, C. H. Wetzel et al., Journal of Neuroscience 19, 7426-7433 (1999)) aktiviert, entspricht allen oben genannte Anforderungen für die hOR17-4-Aktivierung, hat aber benachbart dem aromatischen Ring eine Methylendioxygruppe. Helional (500 µM) führte zu keiner Rezeptoraktivierung, während Canthoxal, ein strukturell ähnlicher Geruchsstoff, dem ein Sauerstoffatom fehlt, klare Ca²⁺-Antworten induzierte.

Bei Betrachtung der aktiven wie auch der inaktiven Geruchsstoffe (vergleiche Fig. 6) ergibt sich eine Anzahl von wichtigen Erfordernissen für einen effektiven hOR17-4-Agonisten: Der Agonist muss eine Aldehydgruppe besitzen. Die Aldehydgruppe muss mit einem aromatischen Rest über eine Kette von 1 bis 3 Atomen verbunden sein. Zumindest eines der Atome der verbindenen Kette kann Sauerstoff sein, die übrigen Atome können insbesondere Kohlenstoff sein. Das Vorhandensein oder Fehlen von unpolaren Methylsubstituenten an der verbindenden Kette wird weitgehend toleriert, während Substanzen mit polaren Strukturen und/oder Doppelbindungen in der Kette den Rezeptor nicht aktivieren.

### Beispiel 12: Klassifizierung der Effizienz der Agonisten

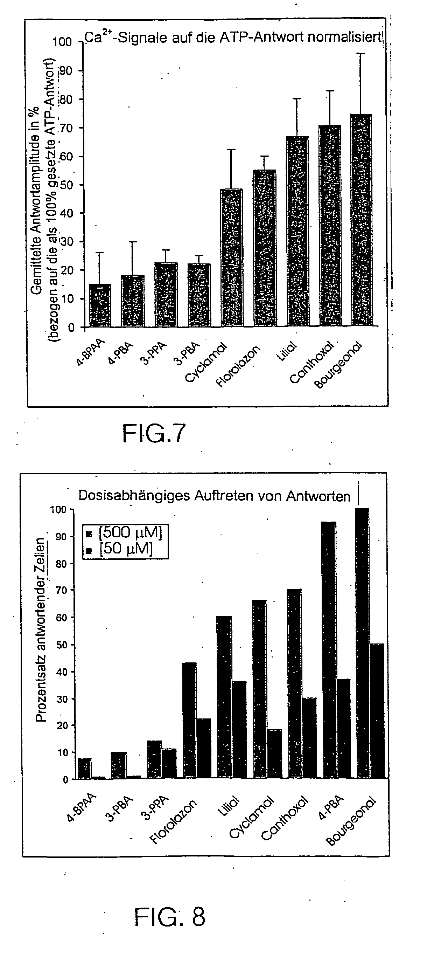

Aufgrund der langanhaltenden Desensitisierung nach der Applikation von Agonisten war es nicht möglich, die Aktivierungswirksamkeit verschiedener hOR17-4-Agonisten in derselben HEK293-Zelle durch deren sukzessive Applikation zu bestimmen. Stattdessen wurde die Wirksamkeit von Geruchsstoffen dem Rang nach durch Normalisieren der gemittelten Antwortamplitude auf das jeweilige ATP-induzierte Ca²⁺-Signal bestimmt. Fig. 7 zeigt diese Rangordnung. Agonisten ohne Substituenten in der para-Position lösten bei weitem die geringsten Antwort-Amplituden (n≥15) aus (18 - 23 % der gemittelten Amplitude der ATP-Antwort, was auf geringe Wirksamkeit hinweist). Cyclamal ist nicht der wirksamste Agonist. Bourgeonal wie auch Canthoxal und Lilial sind wirksamere Agonisten.

Es wurde auch der Vergleich von agonistenabhängigem Auftreten von Zellantworten gegenüber der maximalen Anzahl von potentiell aktivierbaren Zellen (hier definiert als die Zahl der Zellen, die bei sukzessiver Applikation von Cyclamal in außergewöhnlich hoher Dosierung (ca. 5 mM) eine Antwort erzeugten, vergleiche Fig. 8, n ≥ 35) aufgestellt. Die daraus abgeleitete Affinitätsreihenfolge zeigt keine statistisch signifikanten Unterschiede zu der Wirksamkeitsklassifikation, die durch Normalisierung auf die ATP-induzierte Antwort gewonnen wurde. In beiden Fällen war Bourgeonal der wirksamste getestete Agonist. Eine Verringerung der Konzentration der einzelnen Agonisten führte zu einer Verringerung des Prozentsatzes der aktivierten Zellen. Bei der Verringerung der Agonistenkonzentration auf 10⁻⁶ M war nur Bourgeonal deutlich und, zu einem gewissen Ausmaß, Canthoxal in der Lage, eine Ca²⁺-Konzentrationssteigerung zu induzieren. Im Gegensatz dazu erreichte 3-PBA bereits bei ungefähr 50 µM den eine Ca²⁺-Reaktion nicht mehr auslösenden Schwellenwert.

### Beispiel 13: Identifizierung eines Antagonisten

Obwohl Bourgeonal in einer untersuchten Teilmischung ("Mix D") der Henkel-100-Mischung enthalten war, bewirkte die Applikation dieser Teilmischung auf transfizierte HEK293-Zellen keine Erhöhung der cytosolischen Ca²⁺-Konzentration (vergleiche Fig. 9). Daraufhin wurde jede einzelne Komponente von Mix D auf ihre inhibitorischen Eigenschaften untersucht. Es stellte sich heraus, dass die hOR17-4-Rezeptoraktivierung in 26 von 28 Zellen, die auf Bourgeonal antworteten, durch 30 Sekunden oder länger dauernde Vorinkubation mit n-Undecanal vollständig inhibiert wurde (vergleiche Fig. 10). Alle anderen Komponenten von Mix D beeinflussten die Bourgeonal-Sensitivität nicht, vergleiche Fig.11. Es ist möglich, dass n-Undecanal mit nur einer Aminosäureseitenkette der vermuteten Bindungstasche reagiert und dass diese Reaktion nicht ausreicht, den Rezeptor zu aktivieren. Ebenfalls möglich ist es, dass n-Undecanal nicht in der vermuteten Bindungstasche, sondern an einer anderen Stelle bindet und dabei eine Konformationsänderung des Rezeptors bewirkt, die das Binden eines Agonisten in der vermuteten Bindungstasche erschwert.

### Beispiel 14: Überprüfung, ob die gemessenen Ergebnisse repräsentativ für das native Protein hOR17-4 sind

Um zu überprüfen, ob die oben genannten Antworten tatsächlich repräsentativ für das korrespondierende native (nicht-chimäre) Rezeptorprotein hOR17-4 sind oder nur für das chimäre Konstrukt gelten, wurde der Rezeptor hOR17-4 in seiner vollen Länge wie in Beispiel 3 beschrieben kloniert, wie in Beispiel 4 beschrieben transfiziert und nachfolgend exprimiert und auf seine Sensitivität gegenüber Bourgeonal wie auch auf den inhibitorischen Effekt von n-Undecanal untersucht.

Bourgeonal aktivierte deutlich den vollständigen Rezeptor hOR17-4 (vergleiche Fig. 12). Diese spezifische Antwort konnte durch eine 30 bis 60 Sekunden dauernde Vorinkubation der Zellen mit n-Undecanal inhibiert werden (vergleiche Fig. 13).

Dementsprechend ist die Agonistenpezifität kein Artefakt des chimären Proteins. Zudem ist der Bereich der vermuteten Transmembrandomänen III bis VI maßgeblich für die Agonistenbindung. Ferner ist die Bindungsstelle für n-Undecanal auch innerhalb des Bereichs der vermuteten Transmembrandomänen II-VII angeordnet.

Um darüber hinaus zu überprüfen, ob die Aktivierung bzw. der inhibitorische Effekt von Bourgeonal bzw. n-Undecanal hOR17-4-spezifisch ist oder auch in anderen Geruchsrezeptoren auftritt, wurde der früher beschriebene Rezeptor hOR17-40 auf seine Sensitivität gegenüber Bourgeonal und n-Undecanal getestet. Bourgeonal indizierte keine Ca²⁺-Signale (vergleiche Fig. 14), n-Undecanal zeigte keinen inhibitorischen Effekt auf das Helional-induzierte Antwortmuster (vergleiche Fig. 15). Dies weist darauf hin, dass sowohl Bourgeonal als auch n-Undecanal spezifisch hOR17-4 beeinflussen.

### Beispiel 15: Auswirkung von hOR17-4-Agonisten und -Antagonisten auf humane Spermien

Die mögliche Rolle von hOR17-4 in einem Ca²⁺-vermittelten physiologischen Prozess in humanen Spermatozoen (z.B. Acrosomreaktion, Kapazitationsprozess oder Änderungen zur hyperaktivierten Beweglichkeit) wurden durch Ca²⁺-Imaging der Kopf- und der Mittelregion von einzelnen humanen Spermien untersucht. Dazu wurden frisch von jungen, gesunden Spendern erhaltene humane Spermien unverzüglich in 1,5-ml Plastikröhrchen transferiert. Nach 30 minütiger aufrechter Inkubation bei 37 °C wurden 75 µl beweglicher Spermien aus der oberen Schicht der Spermiensuspension auf Concanavalin A-beschichtete 35 mm Petrischalen transferiert und 45 Minuten lang bei Raumtemperatur in 1 ml Ladungspuffer (pH 7,4) mit 140 mM NaCl, 5 mM KCI, 2 mM CaCl₂, 2 mM MgCl₂, 10 mM Hepes, 10 mM Glucose, 5 µM Fura-2-AM (Molecular Probes), and 0,1 % Pluronic F-127 inkubiert. Danach wurde das Medium sanft durch Fura-2-AM-freie Pufferlösung ausgewechselt. Nicht an den Boden angeheftetes Sperma wurde durch diesen Schritt entfernt. Ca²⁺-Bilder wurden von 8 bis 15 Spermatozoen in einem zufällig gewählten Sichtfeld aufgenommen. Die Fluoreszenzsignale eines Spermienkopfes und der Mittelregion wurden gemessen. Um intakte Zellen auszuwählen, wurden nur Spermatozoen in die Analyse eingeschlossen, deren Köpfe angeheftet waren und deren Schwänze schlugen.

Bourgeonal und andere hOR17-4-Agonisten (Aplikationszeit 5 s) induzierten vorübergehende Ca²⁺-Antworten in 36 % aller getesteten Spermatozoen (43 von 119 Spermatozoen, in 12 Experimenten). Diese Ca²⁺-Antworten waren am ausgeprägtesten in der postacrosomalen Region. Die bourgeonalinduzierten Ca²⁺-Signale erreichten ein Maximum nach 15 bis 25 Sekunden nach der Applikation und fielen nach 45 bis 55 Sekunden fast auf den basalen Wert zurück. Anders als bei den rekombinant exprimierten hOR17-4-Rezeptoren war die Aktivierung durch Bourgeonal und anderen Agonisten mehrfach wiederholbar. Lang anhaltende Applikation von Bourgeonal führte zu aufrechterhaltenen, Stimulus-korrelierenden Ca²⁺-Signalen (vergleiche Fig. 16, Applikationsbalken zeigt Aplikation an, die länger als 5 s war).

Der Prozentsatz responsiver Spermatozoen variierte abhängig von der jeweiligen Spermaprobe. Diese Variabilität ist vermutlich auf unterschiedliche Kapazitationszustände der verschiedenen Spermaproben *in vitro* oder auf eine unstabile Expression von hOR17-4 zurückzuführen.

Fig. 17 zeigt den Prozentsatz antwortender Spermatozoen als Funktion der Bourgeonalkonzentration (d.h. eine Dosis-Antwort-Beziehung). Diese Daten zeigen, dass Spermatozoen in der Lage sind, einen Agonisten bei sehr geringen Konzentrationen (Schwellenwert ≥ 10⁻⁸ M, d.h. ungefähr zwei Größenordnungen kleiner als für den rekombinanten Rezeptor) zu detektieren.

Der inhibitorische Effekt von n-Undecanal konnte sowohl im heterologen Expressionssystem als auch in Spermatozoen beobachtet werden. Die Co-Applikation von n-Undecanal in gleicher Konzentration wie Bourgeonal (mit oder ohne n-Undecanal-Vorinkubation) blockierte vollständig die Ca²⁺-Antworten in humanen Spermien (vergleiche Fig. 18; n = 10; 3 unabhängige Experimente).

### Beispiel 16: Einordnung der Effizienz der Agonisten bei Spermazellen

Da anders als bei der heterologen hOR17-4-Expression das Ca²⁺-Imaging im Sperma wiederholte Antworten zeigte, bestand die Möglichkeit, die Effizienz verschiedener hOR17-4 Agonisten direkt zu vergleichen (vergleiche Fig. 19). Die abgeleitete Reihenfolge ist gleich der, die aus der heterologen Expression abgeleitet wurde, mit Bourgeonal als dem potentesten getesteten Liganden.

### Beispiel 17: Physiologische Auswirkungen von Bourgeonal auf humane Spermien

Gefrorene Samenproben von fünf gesunden Spendern wurden aus der California Cryobank (Los Angeles, CA, USA) erhalten und für Bioassays gemäß den World Health Organization-Richtlinien (World Health Organization: Laboratory for the examination of human serum and semen-cervical mucus interaction, Cambridge University Press (1993)) vorbereitet. Nachdem die Suspensionen auf 37 °C für 10 Minuten gebracht worden waren, wurde jede Probe in HEPES-gepufferter humaner Tubarflüssigkeit (HTF; Irvine Scientific, Santa Ana, CA, USA) überführt und auf 10⁷ Zellen/ml eingestellt. Die Lösungen wurden dann bei 37 °C in einer 5 %-igen CO₂-Atmosphäre aufbewahrt und innerhalb von 30 Minuten für Experimente verwendet (P. Quinn, Journal of Assisted Reproductive Genetics 12, 97-105 (1995)). Die Reaktionen von Sperma auf Bourgeonal wurde (a) durch computer-assisted video motion analysis (CAVMA) gemessen, um Veränderungen in der Schwimmgeschwindigkeit (Spermaaktivierung) zu detektieren, und (b) in einem flat capillary-Assay für die Chemotaxis in der Nähe einer Diffusionsmaterialquelle untersucht. Für jede Test-oder Kontroll-Behandlung wurden fünf wiederholte Experimente unter Verwendung von Sperma der fünf Spender durchgeführt. Die Daten der Bioassays wurden statistisch durch Ein-Weg-Varianzanalyse (ANOVA) unter Verwendung eines post-hoc Scheffe-Tests (R. W. Day and G. P. Quinn, Ecol. Monogr. 59, 433-463 (1989)) analysiert. Eine Bonferroni-Korrektur wurde zum Angleichen der α-Levels für multiple Vergleiche verwendet.

Zur Untersuchung der Spermien-Aktivierung wurden Bioassays für die Schwimmgeschwindigkeit bei 37 °C durchgeführt. Spermien wurden sanft einer Kontroll- oder Testlösung einheitlicher Konzentration zugefügt. Die Bilder von Spermien (3,0 x 10³ Zellen/ml) wurden dann unter Verwendung einer Videokamera (NEC Modell TI 23A), die auf einem Olympus IX70-Lichtmikroskop montiert war, bei 90-facher Vergrößerung aufgenommen. Um mögliche Artefakte durch Reibungskräfte zu minimieren, besaß die Kamera eine 100 µm Feldtiefe und fokussierte auf eine Region, die annäherend 2 mm von der nächsten Oberfläche entfernt war. Videobilder von Spermien wurden mit 30 Bildern/Sekunde digitalisiert und über 10-Sekundenintervalle unter Verwendung eines CAVMA-Systemes (Motion Analysis Corporation, Modell VP 320, Expert Vision und Benutzter-Software), verbunden mit einer Sun SPARC 2 Computer-Arbeitsstation bearbeitet (J. A. Riffell, P. J. Krug, R. K. Zimmer, Journal of Experimental Biology 205, 1439-1450 (2002)). Die Schwimmgeschwindigkeit wurde auf einer Bild-zu-Bild-Basis bestimmt und danach über jeden einzelnen zurückgelegten Weg gemittelt. Um zu vermeiden, sich horizontal anstatt vertikal bewegende Zellen zu messen, wurden kurze Wege mit weniger als 11 Bildern verworfen, bei denen sich die Spermien um mehr als 20 % in ihrer scheinbaren Größe veränderten.

Zur Untersuchung der Spermien-Chemotaxis wurden Kammern mit vier getrennten Kompartimenten verwendet, wobei die Kapillar-Methode von Adler (J. Adler, Science 153, 708-716 (1966); J. Adler, Journal of General Microbiology 74, 77-91 (1973)) modifiziert wurde. Jedes Kompartiment bestand aus zwei durch einen Kanal verbundenen Vertiefungen. Die Spermienproben (10⁶ Zellen/ml) wurden jeweils in den Vertiefungen vorgelegt. Ein flaches 6 µl-Mikrokapillarröhrchen (Drummond Scientific Co.), das entweder Test- oder Kontrolllösung enthielt, wurde in den Kanal eingeführt, wobei seine beiden Enden die frischen Spermien-Suspensionen innerhalb der zwei Vertiefungen berührten. Nach vier Stunden Inkubation bei 37 °C wurden die Inhalte jeder Kapillare in die Vertiefung eines Toxoplasmose-Objektträgers transferiert, hitzefixiert und mit 0,1 Acridin-Orange 1 Minute lang gefärbt. Zellzählungen wurden dann unter Verwendung eines Olympus-BH2-Mikroskopes, das für Phasenkontrast- und Epifluoreszenz-Applikationen ausgerüstet war (J. A. Riffell, P. J. Krug, R. K. Zimmer, Journal of Experimental Biology 205, 1439-1450 (2002); C. C. Gee and R. K. Zimmer-Faust, Journal of Experimental Biology 200, 3185-3192 (1997)), bei 67-facher Vergrößerung durchgeführt.

Zur Bestimmung der Chemoattraktion von Spermien und des Effekts eines Antagonisten wurde Bourgeonal allein bei 10⁻⁷ M und in Kombination mit 10⁻⁶, 10⁻⁷, 10⁻⁸ oder 10⁻⁹ M n-Undecanal getestet. Während der Durchführung der Chemotaxis-Bioassays wurde das Spermienverhalten innerhalb eines Bereiches von 300 µm vor jeder Kapillarspitze 30 Sekunden lang auf Video aufgenommen. Die Videoaufnahme begann 10 bis 15 Minuten nach dem Experimentbeginn, um die Bildung eines chemischen Gradienten zu ermöglichen. Die Spermien-Orientierung zum Gradienten hin wurde unter Verwendung von CAVMA quantifiziert. Durch Anwendung zirkulärer Statistiken wurde die mittlere Vektorlänge (r) und die Schwimmrichtung berechnet, um das Bewegungsmuster der untersuchten Spermien zu beschreiben. Der Winkel der Spermienorientierung wurde in Bezug auf einen Ursprung angegeben, der als die kürzeste Verbindung zwischen jeder individuellen Zelle und der Kapillarenspitze (0°) definiert wurde. Um zu bestimmen, ob die Zellbewegung innerhalb des chemischen Gradienten nicht-zufällig war, wurde jede mittlere Ausrichtung mit einer uniformen zirkulären Verteilung unter Verwendung eines unimodalen Rayleigh-Tests (J. H. Zar, Biostatistical Analysis (1984)) verglichen.

Die Spermien reagierten auf Bourgeonal in einer Dosis-abhängigen Weise, indem sie erhöhte Schwimmgeschwindigkeiten und Chemotaxis in Kapillar-Assays zeigten. Eine Konzentration von mehr als 10⁻⁶ M bewirkte keinen weiteren Anstieg der Schwimmgeschwindigkeit oder Zelldichte. Die Figuren 20 und 21 stellen diese Ergebnisse dar, wobei die Werte Mittelwerte und Standartabweichung mit aus den Rohdaten berechneten Regressionsgeraden sind. Fig. 20 zeigt die Schwimmgeschwindigkeit und Fig. 21 die Dichte der Spermien in Abhängigkeit der Konzentration von Bourgeonal (logarithmische Achsenskala).

Bei allen getesteten Konzentrationen von 10⁻⁸ M oder höher wurden signifikante Antworten im Verhältnis zu der HTF-Kontrolle (Ein-Weg-ANOVA: Schwimmgeschwindigkeit: F₆,₁₅₆ = 12,9, P < 0,0001, Scheffé-Test, P ≤ 0,03, alle Vergleiche; Zelldichte: F_{6,204} = 119,9, P < 0,0001, Scheffé-Test, P ≤ 0,03, alle Vergleiche) festgestellt. Die ED₅₀ (Konzentration, die die halbmaximale Antwort hervorruft) war 3 x 10⁻⁸ M sowohl für die Schwimmgeschwindigkeit als auch für die Zelldichte. Die Dosisveränderung hatte daher einen vergleichbaren Effekt auf beide Aspekte des Spermienverhaltens.

Darüber hinaus war Bourgeonal ein bemerkenswert effektiver Chemoattraktant, sogar in sehr verdünnten Konzentrationen. Die Computeranalyse der Spermien-Video-Bilder in der Nähe der Kapillarspitzen bestätigte diese Ergebnisse. Die Zellbewegungsmuster waren signifikant nicht-zufällig und in Antwort auf Bourgeonal-Konzentrationen von 10⁻⁸ M und höher zu den Kapillarspitzen hin ausgerichtet (vergleiche Fig. 24, Rayleigh-Test: r ≥ 0,45, z ≥ 4,42, P ≤ 0,02, alle Vergleiche; Videoaufnahme für 30 Sekunden, alle 0,033 Sekunden eine Aufnahene, die Pfeilköpfe zeigen die Schwimmrichtung der einzelnen Zellen an, Applikation von Bourgeonal in der jeweils angegebenen Konzentration).

Die Figur 22 zeigt die Änderung der Schwimmgeschwindigkeit der Spermien und Fig. 23 die Änderung der Spermien-Zelldichte in den Mikrokapillaren bei Applikation von n-Undecanal, Bourgeonal und Mischungen beider Komponenten.

Die Applikation von 10⁻⁷ M n-Undecanal alleine hatte keinen messbaren Effekt auf das Spermienverhalten (Scheffe-Test: P > 0,99, beide Vergleiche). Relativ zur HTF-Kontrolle war die Schwimmgeschwindigkeit und die Zelldichte als Antwort auf 10⁻⁷ M Bourgeonal nicht signifikant erhöht, wenn dieses mit 10⁻⁶, 10⁻⁷ oder 10⁻⁸ M n-Undecanal (Scheffe-Test: P > 0,99, alle Vergleiche) zusammen appliziert wurde. Dementsprechend ist n-Undecanal ein starker Inhibitor der Wirkung von Bourgeonal auf Spermien (vergleiche Fig. 22, 23 und 24. Fig. 24: Ein-Weg ANOVAs; Schwimmgeschwindigkeit: F_{6,180} = 14,0, P < 0,0001, Scheffé-Test, P < 0,0001, Zelldichte: F_{6,133} = 59,4, P< 0,0001, Scheffé-Test, P < 0,0001; Jeder polare Plot zeigt den mittleren Vektorwinkel der Schwimmrichtung der Spermien gegenüber der Kapillarenspitze (0°); der Pfeil repräsentiert die mittlere Länge des Vektors (r); die Kreise korrespondieren mit in Intervallen von 0,033 Sekunden aufgenommenen Videobildern, die Pfeilspitzen zeigen die Bewegungsrichtungen idividueller Zellen).

### Beispiel 18: Eigenschaften des durch hOR17-4 beeinflussten Signaltransduktionswegs

Gefrorene Samenproben von fünf gesunden Spendern wurden aus der California Cryobank (Los Angeles, CA, USA) erhalten und für Bioassays gemäß den World Health Organization-Richtlinien (World Health Organization: Laboratory for the examination of human serum and semen-cervical mucus interaction, Cambridge University Press (1993)) vorbereitet (vgl. vorheriges Beispiel und Beispiel 7). Bevor oder während die Spermien mit Bourgeonal (100 nM) behandelt wurden, wurden sie jeweils (a) mit Adenylatcyclase-Inhibitor (MDL-12,330A, 50 µM) und (b) mit einem Phospholipase C-Inhibitor (U73122, 50 µM) behandelt.

Die Behandlung mit dem Adenylatcyclase-Inhibitor MDL-12,330A führte dazu, dass 72% der bourgenonal-responsiven Spermien nicht mehr auf Bourgeonal reagierten (fehlende Chemotaxis und fehlende Änderung der Schwimmgeschwindigkeit), während die Behandlung mit dem Phospholipase C-Inhibitor U73122 keine Änderung des Spermienverhaltens bewirkte.

Demnach scheint hOR17-4 auf einen cAMP-Signaltransduktionsweg einzuwirken.

### Beispiel 19: Wirkung von Agonisten und Antagonisten auf die menschliche Nase

An ca. 100 Probanden wurde die Wirkung des Agonisten (Bourgeonal, "Duft A") und des Antagonisten (n-Undecanal, "Duft B") getestet. Dabei wurde zuerst an Duft A (1:100 verdünnt) gerochen, dann wurde in kurzem zeitlichen Abstand (1 - 3 s) an Duft B (ebenfalls 1:100 verdünnt) gerochen, anschließend wurde direkt wieder an Duft A gerochen. Die Probanden wurden nach ihrem Riecheindruck befragt:
Alle 100 Probanden konnten beim jeweils ersten Riechen spezifisch Duft A und B wahrnehmen und unterscheiden. Ca. 90 der 100 Testpersonen beschrieben, dass beim zweiten Riechen an Duft A kein Dufteindruck ausgelöst wurde. Nach ca. 1 Minute war Duft A bereits wieder riechbar.

## Patentansprüche

1. Rezeptor, umfassend einen Proteinabschnitt mit einer Aminosäuresequenz gemäß SEQ ID NO. 8.

2. Vektor, umfassend einen Nucleinsäure-Abschnitt, der für einen Rezeptor nach Anspruch 1 codiert.

3. Expressionssystem, umfassend eine Zelle, die einen Rezeptor nach Anspruch 1 überexprimiert.

4. Biosensor, umfassend
a) einen Rezeptor mit einem Proteinabschnitt mit einer Aminosäuresequenz gemäß SEQ ID NO. 8 und
b) Mittel zum Nachweisen einer Bindung eines Agonisten an den Rezeptor, wobei der Agonist ausgewählt ist aus der Gruppe bestehend aus Phenylacetaldehyd, 3-Phenylpropanal, 3-Phenylbutyraldehyd, 4-Phenylbutyraldehyd, Canthoxal, Cyclamal, Floralazon, Bourgeonal und (4-tert-Butylphenoxy)acetaldehyd.

5. Nichttherapeutische Verwendung
a) eines Agonisten der Formel 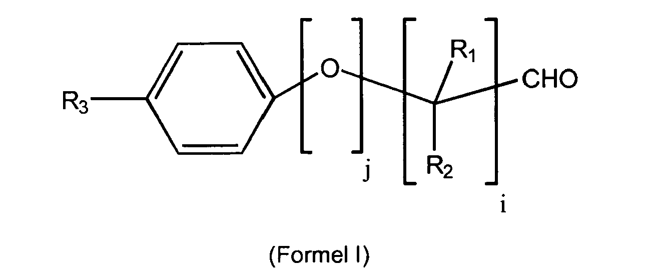 wobei
i: 1, 2 oder 3 ist,
j: 0 oder 1 ist,
jeder der Reste R1, R2 unabhängig von jedem anderen gegebenenfalls vorhandenen Rest R1, R2 -H oder -CH₃ ist und
R3 -H, -CH₃, -C₂H₅, -O-CH₃, -CH₂-CH₃, -CH(CH₃)₂ oder -C(CH₃)₃ ist, und/oder
b) von n-Undecanal,
zum Beeinflussen der Bewegungsrichtung und/oder der Geißelschlagfrequenz eines Spermiums.

6. Verwendung
a) eines Agonisten der Formel 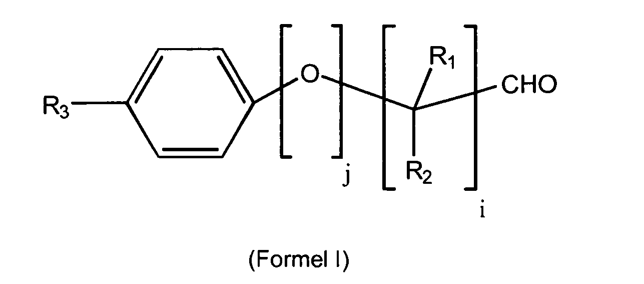 wobei
i: 1, 2 oder 3 ist,
j: 0 oder 1 ist,
jeder der Reste R1, R2 unabhängig von jedem anderen gegebenenfalls vorhandenen Rest R1, R2 -H oder -CH₃ ist und
R3 -H, -CH₃, -C₂H₅, -O-CH₃, -CH₂-CH₃, -CH(CH₃)₂ oder -C(CH₃)₃ ist, und/oder
b) von n-Undecanal,
als Kontrazeptivum oder als Bestandteil eines Kontrazeptivum in einer kontrazeptiven Menge.

7. a) Agonist der Formel 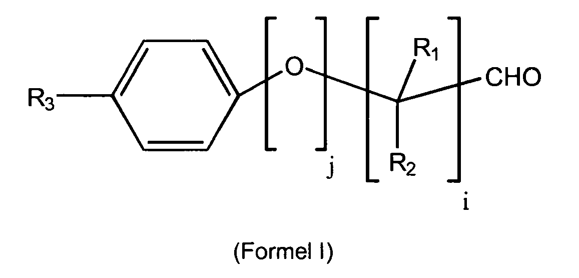 wobei
i: 1, 2 oder 3 ist,
j: 0 oder 1 ist,
jeder der Reste R1, R2 unabhängig von jedem anderen gegebenenfalls vorhandenen Rest R1, R2 -H oder -CH₃ ist und
R3 -H, -CH₃, -C₂H₅, -O-CH₃, -CH₂-CH₃, -CH(CH₃)₂ oder -C(CH₃)₃ ist, und/oder
b) n-Undecanal
zur Anwendung als empfängnisförderndes Arzneimittel oder als Bestandteil eines empfängnisfördernden Arzneimittels in einer empfängnisfördernden Menge.

8. Nichttherapeutische Verwendung von n-Undecanal gegen das spezifische Binden eines Agonisten der Formel 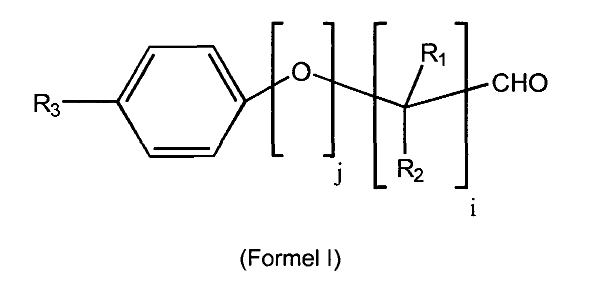 wobei
i: 1, 2 oder 3 ist,
j: 0 oder 1 ist,
jeder der Reste R1, R2 unabhängig von jedem anderen gegebenenfalls vorhandenen Rest R1, R2 -H oder -CH₃ ist und
R3 -H, -CH₃, -C₂H₅, -O-CH₃, -CH₂-CH₃, -CH(CH₃)₂ oder -C(CH₃)₃ ist,
an einen Rezeptor mit einem Proteinabschnitt mit einer Aminosäuresequenz gemäß SEQ ID NO. 4 oder SEQ ID NO. 8, oder mit einem Proteinabschnitt mit einer Aminosäure-Sequenzhomologie von mehr als 70% zu einem Proteinabschnitt mit einer Aminosäuresequenz gemäß SEQ ID NO. 4 oder SEQ ID NO. 8, zum Beeinflussen der Geruchswahrnehmungsfähigkeit eines Menschen und/oder eines Tieres.

9. Verfahren zum Nachweisen eines Antagonisten in einer Probe, umfassend die Schritte:
a) Bereitstellen eines Rezeptors, der einen Proteinabschnitt mit einer Aminosäuresequenz gemäß SEQ ID NO. 4 oder SEQ ID NO. 8, oder mit einem Proteinabschnitt mit einer Aminosäure-Sequenzhomologie von mehr als 70% zu einem Proteinabschnitt mit einer Aminosäuresequenz gemäß SEQ ID NO. 4 oder SEQ ID NO. 8 besitzt,
b) Inkontaktbringen des Rezeptors mit der zu untersuchenden Probe und Einstellen von Bedingungen, bei denen ein gegebenenfalls in der Probe enthaltener Antagonist spezifisch an den Rezeptor bindet,
c) Inkontaktbringen des Rezeptors mit einem Agonisten der Formel (I), um dem Agonisten ein spezifisches Binden an den Rezeptor zu ermöglichen, falls kein Antagonist spezifisch an den Rezeptor gebunden ist, und
d) Überprüfen, ob der Agonist und/oder ein Antagonist eine spezifische Bindung mit dem Rezeptor eingegangen ist.

## Claims

1. Receptor, comprising a protein section with an amino acid sequence according to SEQ ID NO. 8.

2. Vector, comprising a nucleic acid section which codes for a receptor according to claim 1.

3. Expression system, comprising a cell which over-expresses a receptor according to claim 1.

4. Biosensor, comprising
a) a receptor with a protein section with an amino acid sequence according to SEQ ID NO. 8 and
b) means to detect the binding of an agonist to the receptor, wherein the agonist is selected from the group consisting of phenylacetaldehyde, 3-phenylpropanal, 3-phenylbutyraldehyde, 4-phenylbutyraldehyde, canthoxal, cyclamal, floralazon, bourgeonal and (4-tert-butylphenoxy)acetaldehyde.

5. Non-therapeutic use of
a) an agonist of formula 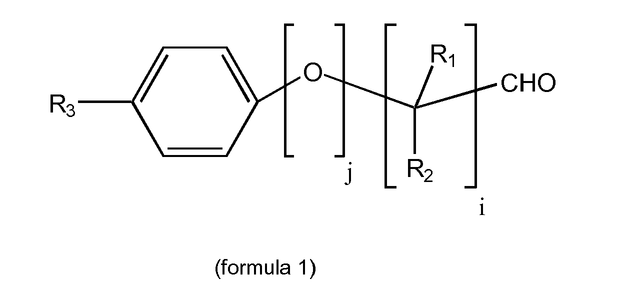 wherein
i: is 1,2 or 3,
j: is 0 or 1,
each residue R1, R2 independent from any other possibly present residue R1, R2 is -H or CH₃ and
R3 is -H, -CH₃, -C₂H₅, -O-CH₃, -CH₂-CH₃, -CH(CH₃)₂ or -C(CH₃)₃,
and/or
b) n-undecanal,
for influencing the direction of movement and/or the frequency of lashing of the flagellum of a sperm.

6. Use of
a) an agonist of formula 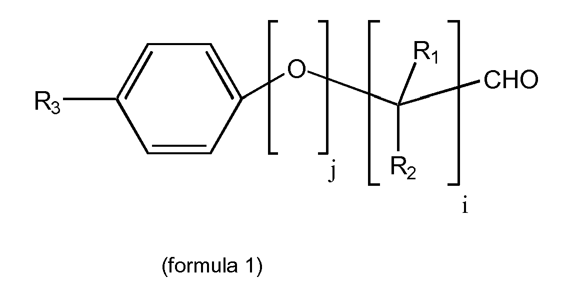 wherein
i: is 1,2 or 3,
j: is 0 or 1,
each residue R1, R2 independent from any other possibly present residue R1, R2 is -H or CH₃ and
R3 is -H, -CH₃, -C₂H₅, -O-CH₃, -CH₂-CH₃, -CH(CH₃)₂ or -C(CH₃)₃,
and/or
b) n-undecanal,
as contraceptive or as a component of a contraceptive in a contraceptive amount.

7. a) Agonist of formula 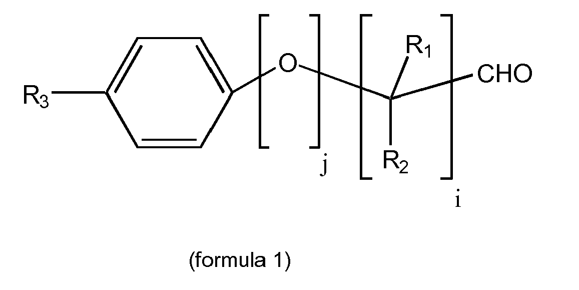
wherein
i: is 1,2 or 3,
j: is 0 or 1,
each residue R1, R2 independent from any other possibly present residue R1, R2 is -H or CH₃ and
R3 is -H, -CH₃, -C₂H₅, -O-CH₃, -CH₂-CH₃, -CH(CH₃)₂ or -C(CH₃)₃,
and/or
b) n-undecanal,
for use as a conception enhancing drug or as component of a conception enhancing drug in a conception enhancing amount.

8. Non-therapeutic use of n-undecanal against the specific binding of an agonist of formula 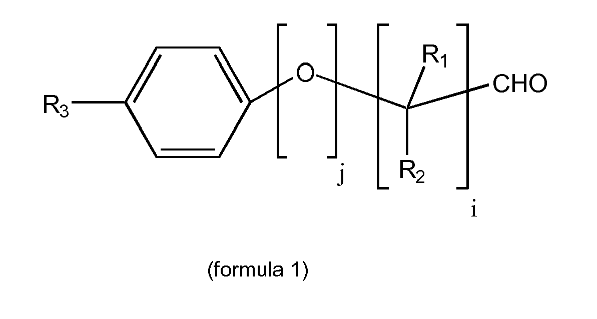 wherein
i: is 1,2 or 3,
j: is 0 or 1,
each residue R1, R2 independent from any other possibly present residue R1, R2 is -H or CH₃ and
R3 is -H, -CH₃, -C₂H₅, -O-CH₃, -CH₂-CH₃, -CH(CH₃)₂ or -C(CH₃)₃,
to a receptor with a protein section with an amino acid sequence according to SEQ ID NO. 4 or SEQ ID NO. 8, or with a protein section with an amino acid sequence homology of more than 70 % to a protein section with an amino acid sequence according to SEQ ID NO. 4 or SEQ ID NO. 8 for influencing the capability of olfactory perception of a human and/or an animal.

9. Method for the detection of an antagonist in a sample, comprising the steps:
a) providing a receptor which has a protein section with an amino acid sequence according to SEQ ID NO. 4 or SEQ ID NO. 8, or with a protein section with an amino acid sequence homology of more than 70 % to a protein section with an amino acid sequence according to SEQ ID NO. 4 or SEQ ID NO. 8,
b) contacting of the receptor with the sample to be tested and adjusting of conditions, in which an antagonist, possibly present in the sample, specifically binds to the receptor,
c) contacting of the receptor with an agonist of formula (I) in order to facilitate a specific binding of the agonist to the receptor in case no antagonist is bound specifically to the receptor, and
d) assessing whether the agonist and/or an antagonist has formed a specific binding to the receptor.

## Revendications

1. Récepteur comprenant une portion de protéine ayant une séquence d'acides aminés selon SEQ ID NO. 8.

2. Vecteur comprenant une portion d'acide nucléique codant pour un récepteur selon la revendication 1.

3. Système d'expression comprenant une cellule surexprimant un récepteur selon la revendication 1.

4. Biocapteur comprenant
a) un récepteur comprenant une portion de protéine ayant une séquence d'acides aminés selon SEQ ID NO. 8, et
b) des moyens de mise en évidence d'une liaison d'un agoniste sur le récepteur, ledit agoniste étant choisi dans le groupe constitué par le phénylacétaldéhyde, le 3-phénylpropanal, le 3-phénylbutyraldéhyde, le 4-phénylbutyraldéhyde, le canthoxal, le cyclamal, le floralazone, le bourgeonal et le (4-tert-butylphénoxy)acétaldéhyde.

5. Utilisation non-thérapeutique
a) d'un agoniste de la formule 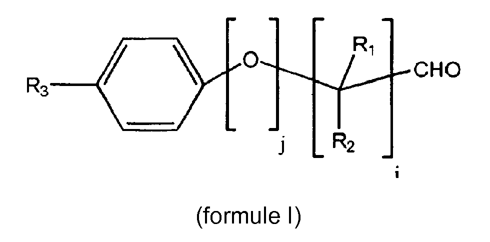 dans laquelle
i est 1, 2 ou 3
j est 0 ou 1,
chacun des restes R1, R2, indépendamment de tout autre reste R1, R2 le cas échéant existant, est -H ou -CH₃, et
R3 est -H, -CH₃, -C₂H₅, -O-CH₃, -CH₂-CH₃, -CH(CH₃)₂ ou -C(CH₃)₃,
et/ou
b) de n-undécanal,
pour influer sur la direction de mouvement et/ou la fréquence de battement du flagelle d'un spermatozoïde.

6. Utilisation
a) d'un agoniste de la formule 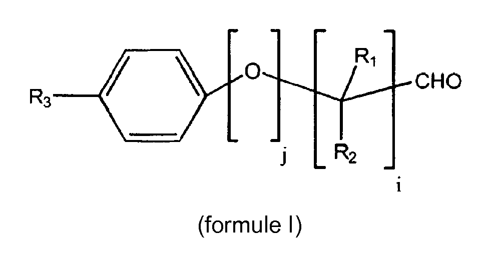 dans laquelle
i est 1, 2 ou 3
j est 0 ou 1,
chacun des restes R1, R2, indépendamment de tout autre reste R1, R2 le cas échéant existant, est -H ou -CH₃, et
R3 est -H, -CH₃, -C₂H₅, -O-CH₃, -CH₂-CH₃, -CH(CH₃)₂ ou -C(CH₃)₃,
et/ou
b) de n-undécanal,
en tant que contraceptif ou en tant que composant d'un contraceptif, en une quantité contraceptive.

7. a) agoniste de la formule 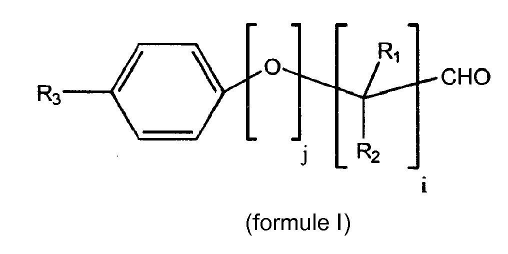
dans laquelle
i est 1, 2 ou 3
j est 0 ou 1,
chacun des restes R1, R2, indépendamment de tout autre reste R1, R2 le cas échéant existant, est -H ou -CH₃, et
R3 est -H, -CH₃, -C₂H₅, -O-CH₃, -CH₂-CH₃, -CH(CH₃)₂ ou -C(CH₃)₃,
et/ou
b) n-undécanal,
pour l'application en tant que médicament favorisant la conception ou en tant que composant d'un médicament favorisant la conception, en une quantité favorisant la conception.

8. Utilisation non-thérapeutique de n-undécanal contre la liaison spécifique d'un agoniste de la formule 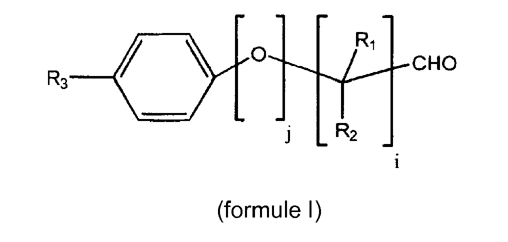 dans laquelle
i est 1, 2 ou 3
j est 0 ou 1,
chacun des restes R1, R2, indépendamment de tout autre reste R1, R2 le cas échéant existant, est -H ou -CH₃, et
R3 est -H, -CH₃, -C₂H₅, -O-CH₃, -CH₂-CH₃, -CH(CH₃)₂ ou -C(CH₃)₃,
sur un récepteur comprenant une portion de protéine ayant une séquence d'acides aminés selon SEQ ID NO. 4 ou SEQ ID NO. 8, ou comprenant une portion de protéine ayant une homologie de séquence d'acides aminés supérieure à 70 % par rapport à une portion de protéine ayant une séquence d'acides aminés selon SEQ ID NO. 4 ou SEQ ID NO. 8, pour influer sur la capacité de perception olfactive d'un être humain et/ou d'un animal.

9. Procédé de mise en évidence d'un antagoniste dans un échantillon, comprenant les étapes consistant à :
a) fournir un récepteur qui possède une portion de protéine ayant une séquence d'acides aminés selon SEQ ID NO. 4 ou SEQ ID NO. 8, ou une portion de protéine ayant une homologie de séquence d'acides aminés supérieure à 70 % par rapport à une portion de protéine ayant une séquence d'acides aminés selon SEQ ID NO. 4 ou SEQ ID NO. 8,
b) mettre en contact ledit récepteur avec l'échantillon à examiner et régler des conditions dans lesquelles un antagoniste contenu le cas échéant dans ledit échantillon se fixe spécifiquement sur le récepteur,
c) mettre en contact le récepteur avec un agoniste de la formule (I) afin de permettre à l'agoniste de se fixer de manière spécifique sur le récepteur, si aucun antagoniste ne s'est fixé spécifiquement sur le récepteur, et
d) vérifier que l'agoniste et/ou un antagoniste est entré en liaison spécifique avec le récepteur.
